# EUROPÄISCHE PATENTSCHRIFT

(11) **EP 2 488 504 B1**
(45) Veröffentlichungstag und Bekanntmachung des Hinweises auf die Patenterteilung: **06.07.2016**
(21) Anmeldenummer: 10754433.0
(22) Anmeldetag: 14.09.2010
(51) Int. Cl.: C07D 249/18, C07D 263/58, A61K 31/4192, A61K 31/423, A61P 35/00

(54) **SULFOXIDEDERIVATE ZUR BEHANDLUNG VON TUMOREN**
SULFOXIDE DERIVATIVES FOR TREATING TUMORS
DÉRIVÉS D'OXYDE SULFONIQUE POUR LE TRAITEMENT DE TUMEURS

(30) Priorität: 13.10.2009 DE 102009049211
(43) Veröffentlichungstag der Anmeldung: 22.08.2012
(73) Patentinhaber: Merck Patent GmbH, 64293 Darmstadt (DE)
(72) Erfinder: SCHIEMANN, Kai, 64342 Seeheim-Jugenheim (DE); SCHULTZ, Melanie, 64287 Darmstadt (DE); STAEHLE, Wolfgang, 55218 Ingelheim (DE)
(86) Internationale Anmeldenummer: PCT/EP2010/005641
(87) Internationale Veröffentlichungsnummer: WO 2011/044978

(56) Entgegenhaltungen:
- WO-A2-2009/046841

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen zur Behandlung von Krankheiten, die mit einer Erhöhung des Lysophosphatsäure Spiegels einhergehen, ferner pharmazeutische Zusammensetzungen, die diese Verbindungen enthalten.

Im einzelnen betrifft die vorliegende Erfindung Verbindungen die bevorzugt eines oder mehrere Enzyme hemmen, die den Lysophosphatsäure (LPS) Spiegel regulieren und/oder modulieren, Zusammensetzungen, die diese Verbindungen enthalten, sowie Verfahren zu ihrer Verwendung zur Behandlung von Krankheiten und Leiden wie Angiogenese, Krebs, Tumorentstehung, -wachstum und -verbreitung, Arteriosklerose, Augenerkrankungen, choroidale Neovaskularisierung und diabetische Retinopathie, Entzündungserkrankungen, Arthritis, Neurodegeneration, Restenose, Wundheilung oder Transplantatabstoßung. Insbesondere eignen sich die erfindungsgemäßen Verbindungen zur Therapie oder Prophylaxe von Krebserkrankungen.

Autotaxin (ATX) ist eine Enzym welches für die Erhöhung des Lysophosphatsäure Spiegel in Ascites und Plasma verantwortlich ist (Xu et al. 1995, Clinical Cancer Research Vol. 1, Seite 1223 und Xu et al. 1995, Biochem. J. Vol- 309, Seite 933). ATX setzt Lysophatidylcholin (LPC) zu Lysophosphatsäure um (Tokumura et al. 2002, J. Biol. Chem., Vol 277, Seite 39436 und Umezu-Gozo et al. 2002, J. Biol. Chem., Vol. 158, Seite 227) LPS ist ein interzellularer Lipid Mediator, der eine Vielzahl von biologischen und biochemischen Prozessen, wie beispeilsweise glatte Muskelkontraktion, Thrombozyten Aggregation und Apoptose beinflusst (Tigyi et al. 2003 Prog. Lipid Res. Vol 42 , Seite. 498 und Mills et al. 2003 Nat. Rev. Cancer Vol. 3, Seite 582 und Lynch et al. 2001 Prost. Lipid Med. Vol.64, Seite 33). Außerdem ist LPS in erhöhten Konzentrationen in Plasma und Ascites Flüssigkeit von Ovariar Krebs Patienten der frühen und späten Phase zu finden. LPA spielt dort eine Rolle bei der Tumorzell Proliferation und deren Invasion in benachbarte Gewebe, welche zur Metastasierung führen kann (Xu et al. 1995, Clinical Cancer Research Vol. 1, Seite 1223 und Xu et al. 1995, Biochem. J. Vol- 309, Seite 933). Diese Prozesse werden durch die Aktivierung durch LPA von G-Protein gekoppelten Rezeptoren angeschaltet (Contos et al. 2000, Mol. Pharm. Vol 58, Seite. 1188).

Aus diesem Grunde ist es zur Behandlung von Tumor Patienten wünschenswert, den LPS Spiegel zu senken. Dies kann durch die Hemmung von Enzymen erreicht werden, die an der LPS Biosynthese beteiligt sind, wie beispielsweise Autotaxin (ATX, Sano et al. 2002, J. Biol. Chem. Vol. 277 , Seite 21197 und Aoki et al. 2003, J. Biol. Chem. Vol. 277 Seite 48737). Autotaxin gehört zu der Enzym Familie der Nucleotide Pyrophosphatasen und Phosphodiesterasen (Goding et al. 1998, Immunol. Rev. Vol. 161, Seite 11) und stellt einen wichtigen Ansatzpunkt bei der antitumoralen Therapie dar (Mills et al. 2003 Nat. Rev. Cancer Vol. 3, Seite 582 and Goto et al. 2004 J. Cell. Biochem. Vol. 92, Seite 1115), da es in Tumoren verstärkte exprimiert wird und Tumorzell Proliferation und deren Invasion in benachbarte Gewebe, welche zur Metastasierung führen kann bewirkt (Nam et al. 2000, Oncogene, Vol. 19 Seite 241). Außerdem bewirkt Autotaxin zusammen mit anderen angiogenetischen Faktoren eine Blugefäßformation im Rahmen der Angiogenese (Nam et al. 2001, Cancer Res. Vol. 61 Seite. 6938). Angiogene ist ein wichtiger Vorgang beim Tumorwachstum, der die Versorgung des Tumors mit Nährstoffen sichert.

Aus diesem Grunde ist die Hemmung der Angiogenes ein wichtiger Ansatzpunkt der Krebs- und Tumortherapie, bei dem der Tumor ausgehungert werden soll (Folkman, 2007, Nature Reviews Drug Discovery Vol. 6, Seite 273-286).

Weiterhin steuert Autotaxin mittels der Umsetzung von LPC zu LPA die Einwanderung von T-Zellen in sekundäre lymphatische Organe. Naive T-Zellen wandern im gesunden Organismus ständig zwischen Blut und sekundären lymphatischen Organen, den Lymphknoten. Um aus dem Blutstrom in einen Lymphknoten einzuwandern, müssen die T-Zellen spezialisierte Blutgefäße, sogenannt High Endothelial Venules (HEV), überwinden. An diesem Prozess ist Autotaxin beteiligt. HEV-Zellen sezernieren Autotaxin in den Blutstrom. Dieses bindet an T-Zellen und setzt auf deren Oberfläche LPC zu LPA um. LPA wiederum bindet an spezifische Rezeptoren auf der Oberfläche der T-Zellen und erhöht deren Fähigkeit, in Lymphknoten einzuwandern. Die Behandlung von T-Zellen mit einer Autotaxin-Mutante, welche enzymatisch inaktiv ist, reduziert deren Fähigkeit, in Lymphknoten einzuwandern (Kanda, H., et al., Autotaxin, an ectoenzyme that produces lysophosphatidic acid, promotes the entry of lymphocytes into secondary lymphoid organs. Nat Immunol, 2008. 9(4): p. 415-23). Die Behandlung der T-Zellen mit den von uns entwickelten Inhibitoren kann ebenfalls deren Migration in Lymphknoten blockieren. Während einer Entzündung können T-Zellen auch in andere Körpergewebe einwandern und dort die Entzündungsreaktion vorantreiben, was zu einer Organschädigung führen kann. Im Tiermodell konnte gezeigt werden, dass Blutgefäße in entzündetem Gewebe beginnen, Autotaxin zu exprimieren [(Nakasaki, T., et al., Involvement of fhe lysophosphatidic acid-generating enzyme autotaxin in lymphocyte-endothelial cell interactions. Am J Pathol, 2008. 173(5): p. 1566-76). Es ist daher anzunehmen, dass Autotaxin während einer Entzündung auch die Einwanderung von T-Zellen in Körpergewebe steuern kann. Tatsächlich findet sich auch im Menschen eine erhöhte Autotaxin-Produktion sowohl in entzündetem Darmgewebe bei chronisch-entzündlichen Darmerkrankungen (Wu, F., et al., Genome-wide gene expression differences in Crohn's disease and ulcerative colitis from endoscopic pinch biopsies: insights into distinctive pathogenesis. Inflamm Bowel Dis, 2007. 13(7): p. 807-21) als auch in betroffenen Gelenken (Nochi, H., et al., Stimulatory role of lysophosphatidic acid in cyclooxygenase-2 induction by synovial fluid of patients with rheumatoid arthritis in fibroblast-like synovial cells. J Immunol, 2008. 181(7): p. 5111-9.) und synovialen Fibroblasten (Kehlen, A., et al., IL-1 beta- and IL-4-induced down-regulation of autotaxin mRNA and PC-1 in fibroblast-like synoviocytes of patients with rheumatoid arthritis (RA). Clin Exp Immunol, 2001. 123(1): p. 147-54.) von Arthritis-Patienten. Da die Einwanderung von T-Zellen in Gewebe bei beiden entzündlichen Erkrankungen eine Rolle spielt, kann die Inhibition von Autotaxin diesen Prozess unterbinden und so einen positiven Einfluss auf den Verlauf der Erkrankung haben.

Es wurde überraschend gefunden, dass die erfindungsgemäßen Verbindungen eine spezifische Inhibierung der Enzym Familie der Nucleotide Pyrophosphatasen und Phosphodiesterasen, insbesondere Autotaxin bewirken. Die erfindungsgemäßen Verbindungen zeigen bevorzugt eine vorteilhafte biologische Aktivität, die in den, zum Beispiel hierin beschrieben Assays, leicht nachweisbar ist. In derartigen Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Generell können alle soliden und nicht soliden Tumore mit den Verbindungen der Formeln Ia bis Im behandelt werden, wie z.B. die Monozytenleukämie, Hirn-, Urogenital-, Lymphsystem-, Magen-, Kehlkopfund Lungenkarzinom, darunter Lungenadenokarzinom und kleinzelliges Lungenkarzinom. Zu weiteren Beispielen zählen Prostata-, Bauchspeicheldrüsen- und Brustkarzinom.

Wie hierin besprochen, sind Wirkungen der erfindungsgemäßen Verbindung für verschiedene Erkrankungen relevant. Dementsprechend sind die erfindungsgemäßen Verbindungen nützlich bei der Prophylaxe und/oder Behandlung von Erkrankungen, die durch eine Inhibierung einer oder mehrerer Nucleotide Pyrophosphatasen und/oder Phosphodiesterasen, insbesondere Autotaxin, beeinflusst werden. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen in einem Xenotransplantat-Tumor-Modell eine vorteilhafte Wirkung aufweisen.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Sensitivität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden können.

### STAND DER TECHNIK

Andere Sulfoxide, die zur Hemmung von Autotaxin fähig sind, sind in WO2009046841 beschieben.
Andere heterocyclische Derivate sind beschrieben in WO 2002085352, WO 2002030422, EP 1002535, WO 9818793, EP 385848, FR 2637286, WO 2005097782, EP 709384, EP 396282, EP 49203.

### ZUSAMMENFASSUNG DER ERFINDUNG

Gegenstand der Erfindung sind die Verbindungen der Formeln Ia bis Im:

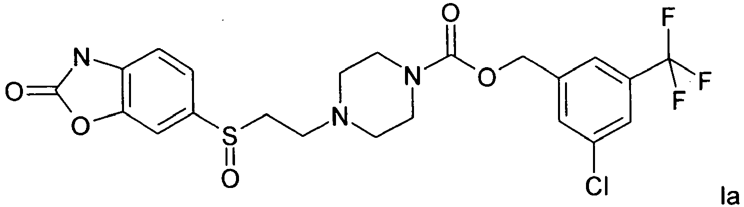

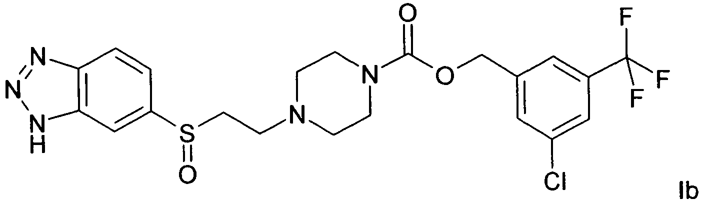

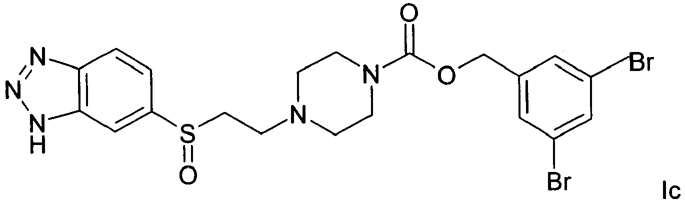

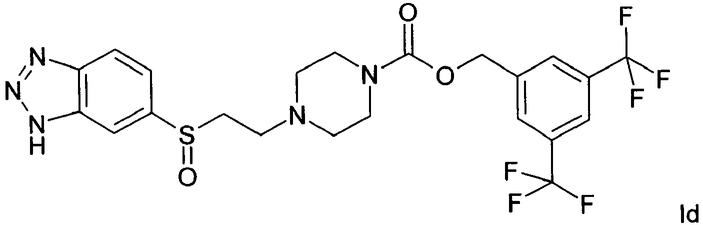

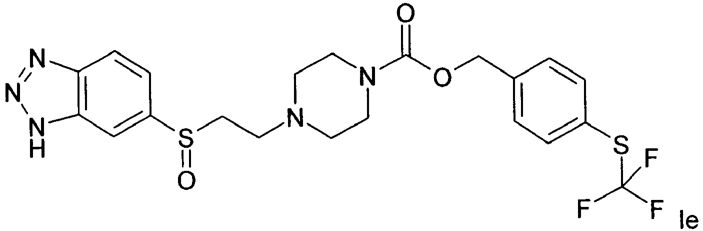

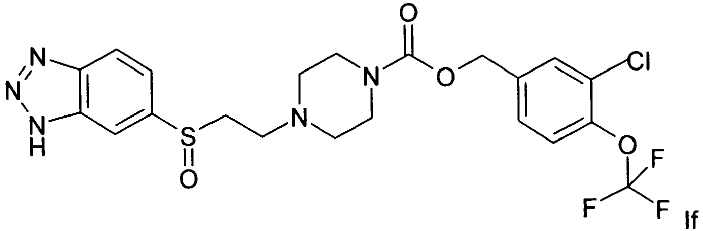

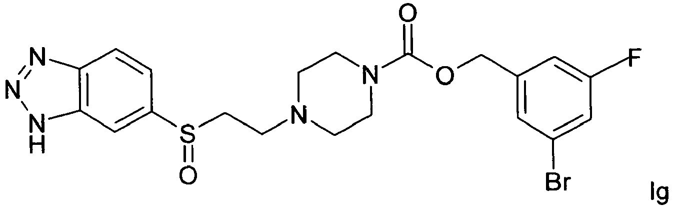

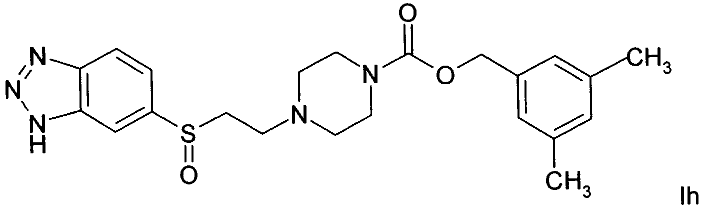

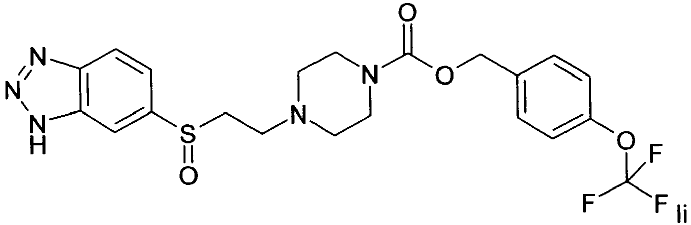

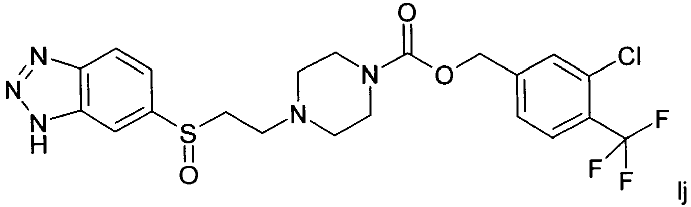

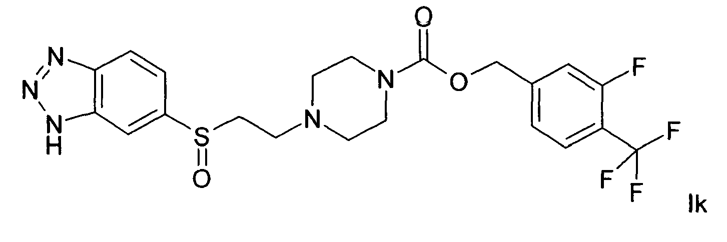

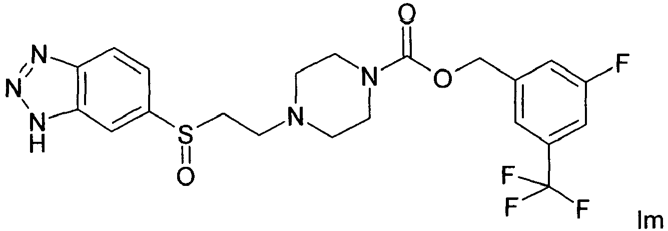

Gegenstand der Erfindung sind auch die optisch aktiven Formen (Stereoisomeren), die Enantiomeren und Tautomere wie im folgenden dargestellt, die Racemate, die Diastereomeren sowie die Salze, Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Die Sulfoxide können auch als bipolare Grenzformel dargestellt werden. Hieraus ergibt sich der Schwefel als Chiralitätszentum und somit das Vorliegen von Enantiomeren:

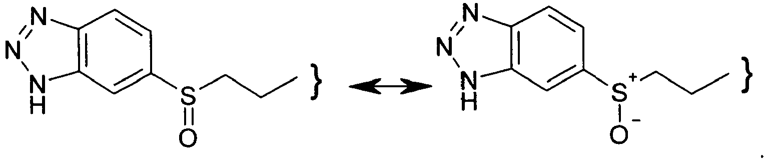

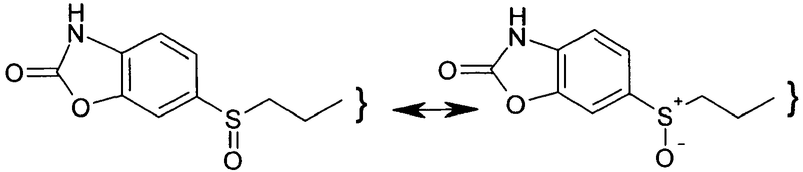

Tautomere:

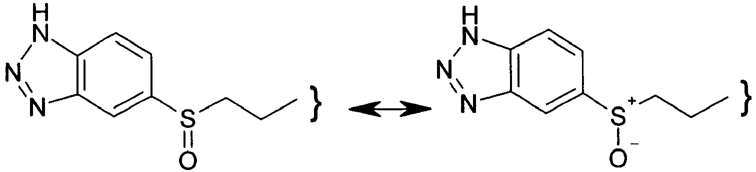

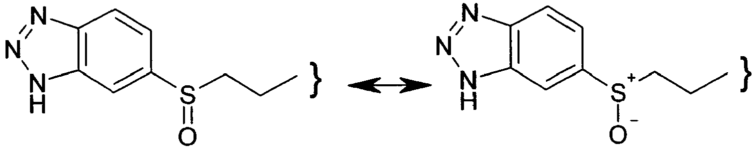

Unter pharmazeutisch verwendbaren Derivaten versteht man z.B. die Salze der erfindungsgemäßen Verbindungen als auch sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfasst auch die Mengen, die wirkungsvoll sind, die normale physiologische Funktion zu erhöhen.

Gegenstand der Erfindung ist auch die Verwendung von Mischungen der erfindungsgemäßen Verbindungen, z.B. Gemische zweier Diastereomerer z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Die Verbindungen der Formel Ia bis Im und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so dass man sie aus dem Reaktionsgemisch nicht isoliert, sondern sofort weiter zu den erfindungsgemäßen Verbindungen Ia bis Im umsetzt.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Acetonitril, Dichlormethan und/oder DMF.

Die genannten erfindungsgemäßen Verbindungen lassen sich in ihrer endgültigen Nichtsalzform verwenden. Andererseits umfasst die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen Ia bis Im werden größtenteils konventionell hergestellt. Sofern die Verbindung der Formel Ia bis Im eine Carbonsäuregruppe enthält, lässt sich eines ihrer geeigneten Salze dadurch bilden, dass man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen Ia bis Im zählen ebenfalls dazu. Bei bestimmten Verbindungen der Formel Ia bis Im lassen sich Säureadditionssalze dadurch bilden, dass man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium, sowie die Erdalkalimetalsalze Calcium und Magnesium. Zu Salzen der Verbindungen Ia bis Im, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formeln Ia bis Im werden dadurch hergestellt, dass man die freie Basenform mit einer ausreichenden Menge der gewünschten Säure in Kontakt bringt, wodurch man auf übliche Weise das Salz darstellt. Die freie Base lässt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formeln Ia bis Im mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, dass man die freie Säureform mit einer ausreichenden Menge der gewünschten Base in Kontakt bringt, wodurch man das Salz auf übliche Weise darstellt. Die freie Säure lässt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfasst die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, dass unter dem Ausdruck "pharmazeutisch unbedenkliches Salz" im vorliegenden Zusammenhang ein Wirkstoff zu verstehen ist, der eine Verbindung der Formeln Ia bis Im in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formeln Ia bis Im und/oder ihre pharmazeutisch verwendbaren und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepasste pharmazeutische Formulierungen können als separate Einheiten, wie z.B. Kapseln oder Tabletten; Pulver oder Granulate; Lösungen oder Suspensionen in wässrigen oder nichtwässrigen Flüssigkeiten; essbare Schäume oder Schaumspeisen; oder Öl-in-Wasser-Flüssigemulsionen oder Wasser-in-Öl-Flüssigemulsionen dargereicht werden.

So lässt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch lässt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unter-schiedlichen Dosierungseinheiten unterscheiden zu können.

Orale Flüssigkeiten, wie z.B. Lösung, Sirupe und Elixiere, können in Form von Dosierungseinheiten hergestellt werden, so dass eine gegebene Quantität eine vorgegebene Menge der Verbindung enthält. Sirupe lassen sich herstellen, indem die Verbindung in einer wässrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung lässt sich auch so herstellen, dass die Freisetzung verlängert oder retardiert wird, wie beispielsweise durch Beschichtung oder Einbettung von partikulärem Material in Polymere, Wachs u.ä.

Die Verbindungen der Formeln Ia bis Im sowie Salze und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel Ia bis Im sowie die Salze und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepasste pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepasste pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepassten pharmazeutischen Formulierungen gehören Augentropfen, wobei der Wirkstoff in einem geeigneten Träger, insbesondere einem wässrigen Lösungsmittel, gelöst oder suspendiert ist.

An die topische Applikation im Mund angepasste pharmazeutische Formulierungen umfassen Lutschtabletten, Pastillen und Mundspülmittel.

An die rektale Verabreichung angepasste pharmazeutische Formulierungen können in Form von Zäpfchen oder Einläufen dargereicht werden.

An die nasale Verabreichung angepasste pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepasste pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepasste pharmazeutische Formulierungen können als Pessare, Tampons, Cremes, Gele, Pasten, Schäume oder Sprayformulierungen dargereicht werden.

Zu den an die parenterale Verabreichung angepassten pharmazeutischen Formulierungen gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, dass die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formeln Ia bis Im hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirk-same Menge einer erfindungsgemäßen Verbindung für die Behandlung von neoplastischem Wachstum, z.B. Dickdarm- oder Brustkarzinom, im Allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so dass die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es lässt sich annehmen, dass ähnliche Dosierungen für die Behandlung der anderen, obenerwähnten Krankheitszustände geeignet sind.

Gegenstand der Erfindung sind ferner Arzneimittel enthaltend mindestens eine Verbindung der Formeln Ia bis Im und/oder Salze, Solvate, Enantiomere, Tautomere und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formeln Ia bis Im und/oder ihrer pharmazeutisch verwendbaren und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formeln Ia bis Im und/oder ihrer pharmazeutisch verwendbaren und Stereoisomere, einschließlich deren Mischungen in allen Verhältnissen, und einer wirksamen Menge eines weiteren Arzneimittelwirkstoffs gelöst oder in lyophilisierter Form vorliegt.

Bevorzugt werden die Verbindungen Ia -Im zur Verwendung in der Behandlung von Tumorerkrankungen wie Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiosarkom, Rhabdomyosarkom, Kolonkarzinom, Pankreaskrebs, Brustkrebs, Ovarkrebs, Prostatakrebs, Plattenzellkarzinom, Basalzellkarzinom, Adenokarzinom, Schweißdrüsenkarzinom, Talgdrüsenkarzinom, Papillarkarzinom, Papillaradenokarzinomen, Cystadenokarzinomen, Knochenmarkkarzinom, bronchogenem Karzinom, Nierenzellkarzinom, Hepatom, Gallengangkarzinom, Chorionkarzinom, Seminom, embryonalem Karzinom, Wilms-Tumor, Cervix-Krebs, Hodentumor, Lungenkarzinom, kleinzelligem Lungenkarzinom, Blasenkarzinom, Epithelkarzinom, Gliom, Astrocytom, Medulloblastom, Kraniopharyngiom, Ependymom, Pinealom, Hämangioblastom, akustischem Neurom, Oligodendrogliom, Meningiom, Melanom, Neuroblastom, Retinoblastom, Leukämie, Lymphom, multiplem Myelom, Waldenströms Makroglobulinämie und Schwere-Kettenerkrankung eingesetzt.

Bevorzugt werden die Verbindungen der Formeln Ia bis Im mit den mit bekannten Antikrebsmitteln kombiniert:
Zu diesen bekannten Antikrebsmitteln zählen die folgenden:
   Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptor modulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den
Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzylidenchartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-hepto-pyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo-(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

Insbesondere bevorzugt ist die Verwendung der erfindungsgemäßen Verbindung zur Behandlung und Prophylaxe von Tumorerkrankungen.

Der Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge.

Der Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Unter einem anderen Aspekt umfasst die Erfindung ein zur Behandlung eines Patienten, der ein Neoplasma, wie einen Krebs, hat, durch Verabreichung einer Verbindung der Formel Ia bis Im in Kombination mit einem antiproliferativen Mittel. Geeignete antiproliferative Mittel umfassen die in Tabelle 1 bereitgestellten.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetet oder Dichlormethan, trennt ab, trocknet die organische Phase über Natriumsulfat, dampft ein und reinigt duch Chromatographie an Kieselgel und/oder durch Kristallisation..
Massenspektrometrie (MS):
EI (Electronenstoß-Ionisation) M⁺
FAB (Fast Atom Bombardment) (M+H)⁺
ESI (Electrospray lonization) (M+H)⁺ APCI-MS (atmospheric pressure chemical ionization - mass spectrometry) **(M+H)⁺**

### Methode LC/MS:

Solvent A: Wasser + 0,1 % TFA
Solvent B: Acetonitril + 0,1 % TFA
Flow: 2,4 ml/min
Gradient:
   0,0 min 4 % B
   2,6 min 100 % B
Säule: Chromolith Speed ROD RP-18e 50-4, 6 mm

Die folgenden Substanzen wurden synthetisiert und charakterisiert. Die Herstellung und Charakterisierung der Substanzen sind jedoch auch auf anderen Wegen für den Fachmann durchführbar.

### Beispiel 1

Synthese von 4-[2-(3H-Benzotriazol-5-(R)sulfinyl)-ethyl]-piperazin-1-carbonsäure-3,5-difluoromethylbenzylester 10a 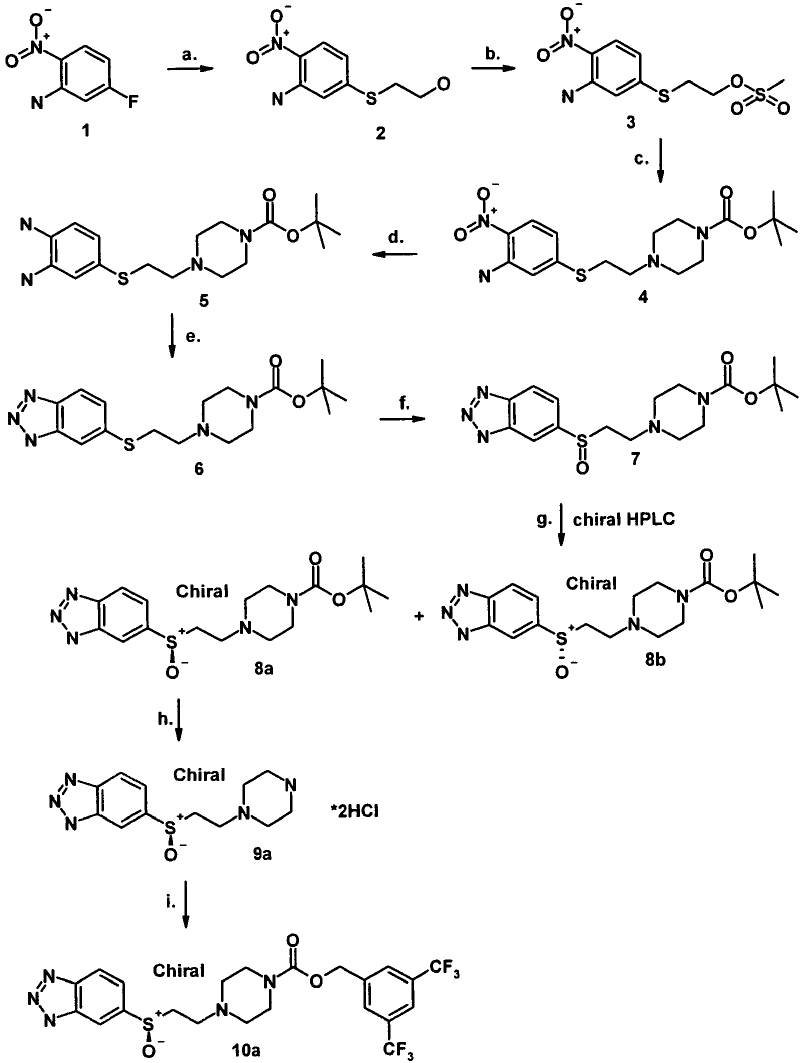
a. 5-Fluoro-2-nitrophenylamin 1 (33,6 g, 213 mmol, 90%ig) und Cäsiumcarbonat (77,1 g, 237 mmol) werden in 400 ml Acetonitril suspendiert (rötliche Suspension). Zu dieser Suspension werde 2-Mercaptoethanol (18,5 g, 237 mmol) bei RT hinzugegeben, wobei sich das Reaktionsgemisch sofort gelb verfärbt. Es wird anschließend 18 h bei 60°C gerührt. Das Gemisch wird filtriert, der Rückstand mit Acetonitril nachgewaschen und das Filtrat bis zur Trockne eingedampft. Man erhält einen gelblich-orangen Feststoff, der sich als die Verbindung 2 herausstellte und ohne weitere Aufreinigung weiter umgesetzt wird.
b. Verbindung 2 (45,5 g, 212 mmol) wird in 250 mL Acetonitril suspendiert. Zu dieser tief roten Suspension wird Triethylamin (22,6 g, 223 mmol) bei RT zugegeben und anschließend 30 min bei RT gerührt. Methansulfonylchlorid (25,5 g, 223 mmol) als Lösung in 20 ml Dichlormethan wird langsam bei RT zugetropft (exotherme Reaktion) und anschließend 3h bei RT weitergerührt, wobei das Produkt als gelber Niederschlag ausfällt. Die Mischung wird 2 h in Eis eingekühlt, der gelbe Niederschlag abfiltriert und mit reichlich Acetonitril nachgewaschen. Filtrat und Waschlauge werden vereint und eingeengt. Die aufkonzentrierte Lösung wird dann ebenfalls eingekühlt und weiteres Produkt mit der doppelten Menge Methyl-tert.Butylether ausgefällt. Diese wird ebenfalls abfiltriert und mit Acetonitril nachgewaschen. Die beiden Filterrückstände werden vereinigt und im Vakuum getrocknet. Man erhält einen gelben Feststoff der Verbindung 3, welcher ohne weitere Aufreinigung weiter umgesetzt wird.
c. Verbindung 3 (61,9 g, 212 mmol) und Boc-piperazin (47,0 g, 250 mmol) werden in 400 ml THF suspendiert. Zu dieser gelben Suspension wird eine Lösung von 25,7g (254 mmol) Triethylamin in 20ml THF langsam zugetropft. Nach vollendeter Zugabe wird das Gemisch 15 h bei 70°C gerührt. Der entstandene Niederschlag wird abfiltriert, mit THF gewaschen und verworfen. Das Filtrat und Waschlauge wird vereint und zur Trockne eingedampft. Dieser Rückstand wird in Ethylacetat aufgenommen, mehrmals mit Wasser und mit NaCl-Lösung gewaschen, mit Natriumsulfat getrocknet, filtriert und zur Trockne eingedampft. Das gelbe Produkt 4 wird ohne weitere Aufreinigung weiter umgesetzt.
d. Verbindung 4 (40,1 g, 105 mmol) wird in 540 mL THF gelöst, mit Sponge-Nickel-Catalyst (wassernass, 10 g) versetzt und 15 h bei Wasserstoff-Normaldruck bei RT geschüttelt. Es werden weitere 10 g des Katalysators zugegeben und weitere 17 h in Wasserstoffatmosphäre geschüttelt. Der Katalysator wird abfiltriert und das Filtrat zur Trockne eingedampft. Dieser schwarze ölige Rückstand wird in Ethylacetat aufgenommen, drei mal mit Wasser und ein mal mit NaCl-Lösung gewaschen, mit Natriumsulfat getrocknet, filtriert und einrotiert. Das dunkelrote Öl der Verbindung 5 wird ohne weitere Aufreinigung weiter verarbeitet.
e. Verbindung 5 (32,5 g, 92,2 mmol) wird in Eisessig (320 mL) gelöst und Natriumnitrit (6.4 g, 92,8 mmol) hinzugegeben. Es wird 2h bei RT gerührt. Dann wird mit Wasser verdünnt und die Reaktionslösung mehrmals mit Ethylacetat extrahiert. Die org. Phase wird mit NaHCO3-Lösung und NaCl-Lösung gewaschen, mit Na2SO4 getrocknet, filtriert und zur Trockne eingedampft. Das dunkle harzige Öl der Verbindung 6 wird ohne weitere Aufreinigung weiter verarbeitet.
f. Verbindung 6 (27,1 g, 74,5 mmol) wird in 320 mL Eisessig gelöst und unter Rühren 15,5 ml Wasserstoffperoxid (30%ig in Wasser) hinzugetropft. Das Gemisch wird bei RT 15 h gerührt, mit Wasser und Ethylacetat verdünnt und dann unter Rühren erst mit festem NaHCO3 dann mit NaHCO3-Lösung neutralisiert. Die organische Phase wird isoliert und mit Wasser und mit NaCl-Lösung gewaschen, mit Natriumsulfat getrocknet, filtriert und zur Trockne eingedampft. Das dunkle amorphe Kristallisat wird als Verbindung 7 identifiziert und ohne weitere Aufreinigung in die Enantiomere aufgetrennt.
g. Verbindung 7 (21,0 g, 44,3 mmol) wird in 250 mL Methanol gelöst. Die Lösung wird an der präparativen SFC (je 8mL pro Aufgabe) über Chiralpak AD-H Säule 3x20cm 5µm und CO2 (80mL) und Methanol (16 mL) getrennt. 2 Fraktionen werden gesammelt und jeweils zur Trockne eingeengt. Fraktion 1 enthält 6,9 g (18.2 mmol, 41%) eines farblosen Feststoffes, welchem willkürlich die absolute Struktur 8b zugeordnet wird. Struktur 8a wird dem farblosen Feststoff der Fraktion 2 zugeordnet.
h. Die enantiomerenreine Verbindung 8a (7,00 g, 18,4 mmol) wird in 80 ml Isopropanol gelöst, unter Rühren 80 ml 5-6N HCl in Isopropanol bei RT hinzugegeben und weitere 15 h bei RT gerührt. Die Mischung wird unter Vakuum zur Trockne eingedampft, in wenig Dioxan gelöst, Wasser zugemischt, eingefroren und lyophylisiert. Man erhält einen farblosen Feststoff hoher Reinheit, der der Verbindung 9a als Dihydrochlorid zugeordnet werden kann. Das Material wird ohne weitere Aufreinigung weiterverwendet.
i. Die Verbindung 9a (175 mg, 0,50 mmol) wird in 2 ml DMF gelöst und das Triethylamin 151 µL, 1,09 mmol) hinzugegeben. 3,5-Bistrifluormethylbenzylalkohol (124 mg, 0,50 mmol, 98%ig) und 1,1'-Carbonyldiimidazol (80,6 mg, 0,50 mmol) werden in einen separatem Gefäß eingewogen, in 3 ml DMF gelöst und eine Stunde bei RT gerührt. Zu dieser Mischung wird die vorher angefertigte Lösung von Verbindung 9a hinzugegeben und die Gesamtlösung über Nacht bei RT gerührt. Das Gemisch wird zur Trockne eingedampft, der Rückstand in Dichlormethan aufgenommen, mit Wasser und NaCl-Lösung gewaschen, mit Natriumsulfat getrocknet, filtriert und erneut zur Trockne eingedampft. Der Rückstand wird säulenchromatographisch aufgereinigt (Ethylacetat/MeOH). Man erhält der Verbindung 10a als farblosen Feststoff.

Analog der beschriebenen Vorschriften h. und i. lässt sich auch das Enantiomer 8b zu dem entsprechenden Antipoden der Verbindung 10a überführen.

Ebenfalls lässt sich das Racemat 7 über die Vorschriften h. und i. in das racemische Gemisch von 10a überführen. Analog g. kann auch auf dieser Stufe die Trennung in die Antipoden erfolgen.

### Beispiel 2

Synthese von 4-[2-(2-Oxo-2,3.dihydro-benzooxazolyl-6(R)-sulfinyl)-ethyl]-piperazin-1-carbonsäure-3-chlor-5-trifluormethylbenzylester 20a 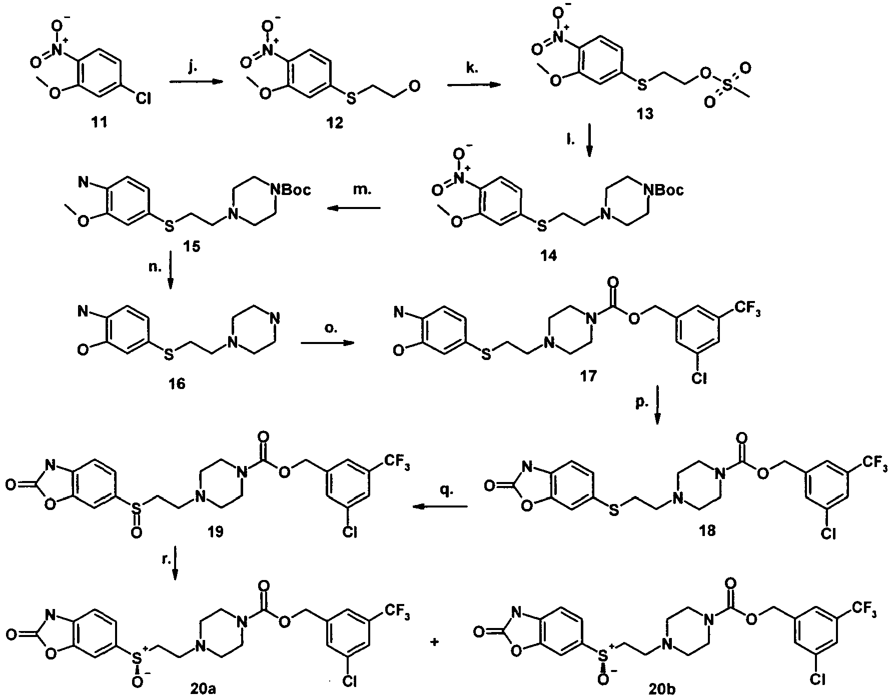
j. 5-Chlor-2-nitroanisol (48,3 g, 0,25 mol) wird in 300 ml Acetonitril gelöst. Man gibt unter Rühren eine Lösung von 2- Mercaptoethanol (17,8 ml, 0,25 mol) in 100 ml Acetonitril und Kaliumcarbonat (69,1 g, 0,5 mol) hinzu und rührt über Nacht am Rückfluss. Das Reaktionsgemisch wird abgekühlt, mit 500 ml Eiswasser versetzt und dann zweimal mit 400 ml Essigester extrahiert. Die organische Phase wird mit 0,2 n NaOH-Lösung und dann mit Wasser gewaschen, über Magnesiumsulfat getrocknet, abfiltriert und am Rotationsverdampfer eingeengt. Durch Kristallisation mit Diethylether erhält man beige Kristalle der Verbindung 12.
k. Verbindung 12 (48,0 g, 0,209 mol) wird in 50 ml Dichlormethan suspendiert und mit Triethylamin (19 ml, 0,209 mol) versetzt. Unter Rühren wird bei maximal 15° C Innentemperatur Methansulfonylchlorid (16,2 ml, 0,209 mol) zugetropft. Man rührt noch eine Stunde bei Raumtemperatur, gibt dann das Reaktionsgemisch auf 300 ml Eiswasser und extrahiert zweimal mit 200 ml Dichlormethan. Die organische Phase wird mit Magnesiumsulfat getrocknet, abfiltriert und am Rotationsverdampfer eingeengt. Man erhält Verbindung 13 als öligen Rückstand.
I. Verbindung 13 (57 g, 0,185 mol), Piperazin-1-carbonsäure-tert.-butylester (34,5 g, 0,185 mol), und Cäsiumcarbonat (60,1 g, 0,185 mol) werden in 300 ml Acetonitril gelöst und bei 60° C über Nacht gerührt. Man versetzt mit 400 ml Wasser und 400 ml Dichlormethan und trennt die organische Phase ab. Sie wird mit Magnesiumsulfat getrocknet, abfiltriert und am Rotationsverdampfer eingeengt. Der Rückstand wird über eine Kieselgelsäule mit Essigester als Laufmittel aufgereinigt. Man erhält Verbindung 14 als amorphe Festsubstanz.
m. Verbindung 14 (6,3 g, 15,8 mmol) wird in 85 ml Tetrahydrofuran mit 3,15 g Pd-C-5% versetzt. Und bei Raumtemperatur 16 Stunden hydriert. Der Katalysator wird abfiltriert. Nach Einengen der Lösung erhält man die Verbindung 15 als Festsubstanz.
n. Verbindung 15 (5,5 g 0,015 mol) werden in 70 ml 47%-iger Bromwasserstoffsäure gelöst und 8 Stunden bei 150° C gerührt. Dann lässt man das Reaktionsgemisch abkühlen und saugt die ausgefallenen Kristalle ab. Sie werden mit etwas Wasser gewaschen und getrocknet. Man erhält die Verbindung 16.
o. Verbindung 16 (1,18 g, 3,0 mmol), 1,1-Carbonyldiimidazol (0,486 g, 3,0 mmol) 3-Chlor-5-trifluormethylbenzylalkohol (0,63 g, 3,0 mmol) und Triethylamin (0,42 ml, 3,0 mmol) werden in 20 ml DMF gelöst und über Nacht bei RT gerührt. Das Reaktionsgemisch wird eingeengt und in 50 ml Wasser und 100 ml Essigester aufgenommen. Die organische Phase wird abgetrennt, mit Magnesiumsulfat getrocknet, abfiltriert und am Rotationsverdampfer eingeengt. Der Rückstand wird über eine Kieselgelsäule mit Essigester als Laufmittel aufgereinigt. Man erhält Verbindung 17 als Festsubstanz.
p. Verbindung 17 (0,8 g, 1,63 mmol) und 1,1-Carbonyldiimidazol (0,265 g, 1,63 mmol) werden in 10 ml THF 3 Stunden bei RT gerührt. Dann wird der Ansatz mit 100 ml Wasser versetzt und zweimal mit 100 ml Essigester extrahiert. Die organische Phase wird mit Magnesiumsulfat getrocknet, abfiltriert und am Rotationsverdampfer eingeengt. Die Kristallisation mit Ethanol ergibt Verbindung 18 als hellbraune Kristalle.
q. Verbindung 18 (0,72 g, 1,4 mmol) wird in 8 ml Eisessig gelöst. Dann gibt man Wasserstoffperoxid (30%-ig in Wasser, 0,29 ml 2,8 mmol) hinzu und rührt 3 Stunden bei RT. Dann gibt man den Ansatz auf 100 ml Eiswasser und neutralisiert mit gesättigter NaHCO3-Lösung. Man extrahiert zweimal mit 50 ml Essigester. Die organische Phase wird mit Magnesiumsulfat getrocknet, abfiltriert und am Rotationsverdampfer eingeengt. Die Kristallisation mit Ethanol ergibt Verbindung 19 als helle Kristalle.
r. Das Racemat 19 (0,35 g, 0,66 mmol) wird mittels präparativer HPLC an Chiralpak AD (5x40 cm, 20 µm) mit Methanol/Ethanol (25/75) getrennt (Fluss: 100 ml/min). Man erhält die beiden Enantiomeren 20a und 20b als amorphe Festsubstanz.

Die nach folgenden Verbindungen können auf analoge Weise wie in den Beispielen 1 und 2 dargestellt, hergestellt werden:
4-[2-(2-Oxo-2,3-dihydro-benzooxazol-6(R)-sulfinyl)-ethyl]-piperazin-1-carbonsäure 3-chloro-5-trifluoromethyl-benzyl ester (la) 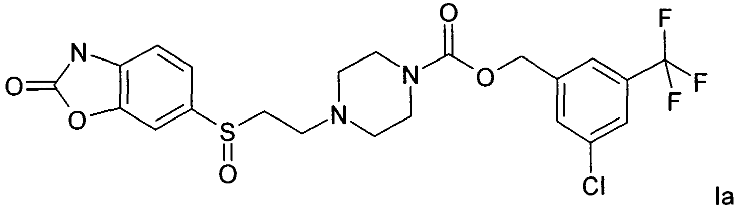 Racemat: 1H NMR (500 MHz, DMSO) δ = 12.02 (m, <1H), 7.81 (s, 1 H), 7.75 (s, 1H), 7.69 (s, 1H), 7.62 (d, J=1.5, 1 H), 7.47 (dd, J=8.1, 1.5, 1H), 7.25 (d, J=8.1, 1H), 5.15 (s, 2H), 4.45 - 3.30 (m, 4H), 3.14 - 3.07 (m, 1H), 3.00 - 2.90 (m, 1 H), 2.75 -2.68 (m, 1 H), 2.47 - 2.27 (m, 5H), Rt[min] 1,80,
4-[2-(3H-Benzotriazol-5(R)-sulfinyl)-ethyl]-piperazin-1-carbonsäure 3-chloro-5-trifluoromethyl-benzyl ester (Ib) 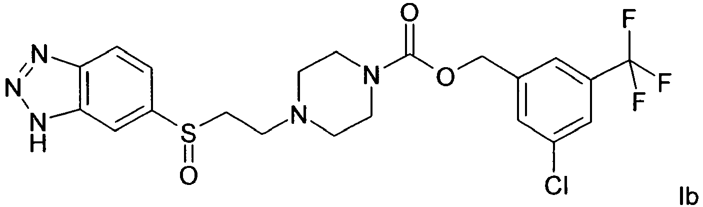 1 H NMR (400 MHz, DMSO) δ 15.87 (s, 1 H), 8.24 (s, 1 H), 8.09 (d, J = 8.7, 1 H), 7.82 (s, 1 H), 7.78 - 7.67 (m, 3H), 5.15 (s, 2H), 3.55 - 3.14 (m, 5H), 3.07 - 2.91 (m, 1 H), 2.81 - 2.72 (1 H), 2.48 - 2.28 (m, 5H), Rt[min] 1,79,
4-[2-(3H-Benzotriazol-5(R)-sulfinyl)-ethyl]-piperazin-1- carbobsäure 3,5-dibromo-benzyl ester (Ic) 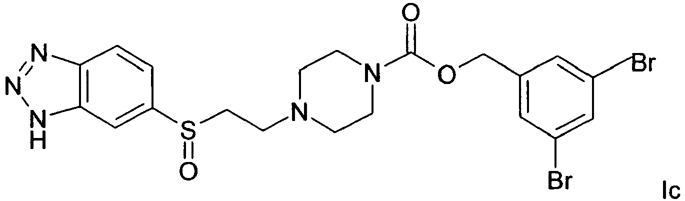 1 H NMR (500 MHz, DMSO) δ = 16.03 (s, 1 H), 8.25 (s, 1 H), 8.10 (d, J=8.6, 1 H), 7.79 (t, J=1.6, 1 H), 7.74 (d, J=8.5, 1 H), 7.58 (d, J=1.6, 2H), 5.06 (s, 2H), 3.52 - 3.17 (m, 5H), 3.06 - 2.96 (m, 1H), 2.81 - 2.74 (m, 1H), 2.50 - 2.28 (m, 5H), Rt[min] 1,76,
4-[2-(3H-Benzotriazol-5(S)-sulfinyl)-ethyl]-piperazin-1-carbonsäure 3,5-bistrifluoromethyl-benzyl ester (Id) 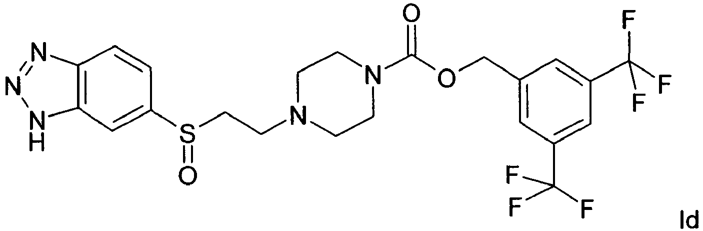 1 H NMR (400 MHz, DMSO) δ = 15.98 (s, 1 H), 8.25 (s, 1 H), 8.15 - 8.00 (m, 4H), 7.74 (d, J=8.6, 1 H), 5.25 (s, 2H), 3.40 - 3.14 (m, 5H), 3.10 - 2.92 (m, 1 H), 2.85 - 2.68 (m, 1H), 2.49 - 2.24 (m, 5H), Rt[min] 1,85,
4-[2-(3H-Benzotriazol-5(R)-sulfinyl)-ethyl]-piperazin-1-carbonsäure 4-trifluoromethylsulfanyl-benzyl ester (Ie) 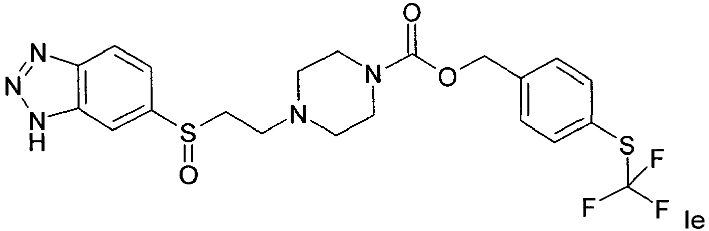 1 H NMR (400 MHz, DMSO) δ = 16.02 (s, 1 H), 8.25 (s, 1 H), 8.10 (d, J=8.6, 1 H), 7.76 - 7.70 (m, 3H), 7.50 (d, J=8.3, 2H), 5.14 (m, 2H), 3.47 - 3.15 (m, 5H), 3.08 - 2.94 (m, 1H), 2.81 - 2.72 (m, 1H), 2.48 - 2.27 (m, 5H), Rt[min] 1,85,
4-[2-(3H-Benzotriazol-5(R)-sulfinyl)-ethyl]-piperazin-1-carbonsäure 3-chloro-4-trifluoromethoxy-benzyl ester (If) 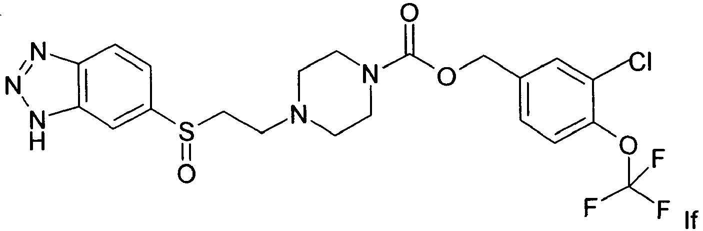 1 H NMR (500 MHz, DMSO) δ = 16.08 (s, 1 H), 8.24 (s, 1 H), 8.09 (d, J=8.6, 1H), 7.73 (dd, J=8.6, 1.1, 1H), 7.67 (d, J=2.0, 1H), 7.57 (dd, J=8.6, 1.1, 1H), 7.45 (dd, J=8.5, 2.0, 1 H), 5.07 (s, 2H), 3.35 - 3.15 (m, 5H), 3.07 - 2.95 (m, 1 H), 2.80 - 2.72 (m, 1 H), 2.48 - 2.30 (m, 5H), Rt[min] 1,80,
4-[2-(2-Oxo-2,3-dihydro-benzooxazol-6(R)-sulfinyl)-ethyl]-piperazin-1-carbonsäure 3-bromo-5-fluoromethyl-benzyl ester 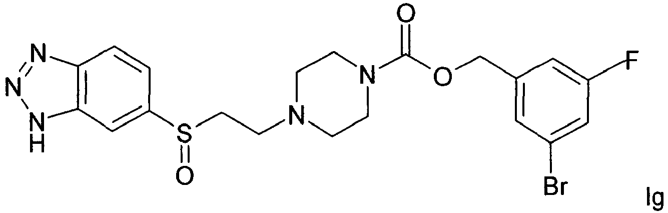 1 H NMR (500 MHz, DMSO) δ = 16.10 (s, 1 H), 8.25 (s, 1 H), 8.10 (d, J=8.6, 1 H), 7.74 (d, J=8.6, 1 H), 7.55 - 7.46 (m, 1 H), 7.42 (s, 1 H), 7.24 (d, J=9.2, 1 H), 5.08 (s, 2H), 3.32 - 3.14 (m, 5H), 3.09 - 2.94 (m, 1 H), 2.81 - 2.74 (m, 1 H), 2.50 - 2.27 (m, 5H), Rt[min] 1,63,
4-[2-(3H-Benzotriazol-5(R)-sulfinyl)-ethyl]-piperazin-1-carbonsäure 3,5-dimethyl-benzyl ester (Ih) 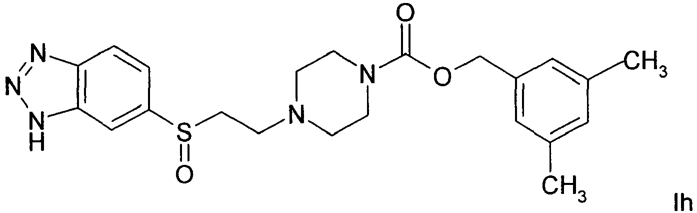 1 H NMR (500 MHz, DMSO) δ = 15.91 (s, 1 H), 8.24 (s, 1 H), 8.09 (d, J=8.6, 1 H), 7.73 (dd, J=8.6, 1.1, 1H), 6.93 (s, 3H), 4.97 (s, 2H), 3.35 - 3.13 (m, 5H), 3.07 - 2.93 (m, 1 H), 2.80 - 2.71 (m, 1 H), 2.50 - 2.29 (m, 6H), 2.25 (s, 5H), Rt[min] 1,62,
4-[2-(3H-Benzotriazol-5(R)-sulfinyl)-ethyl]-piperazin-1-carbonsäure 4-trifluoromethoxy-benzyl ester (Ii) 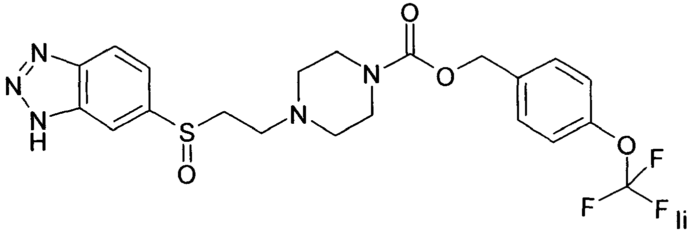 1 H NMR (400 MHz, DMSO) δ = 15.99 (s, 1 H), 8.24 (s, 1 H), 8.09 (d, J=8.6, 1 H), 7.79 - 7.69 (m, 1 H), 7.48 (d, J=8.3, 2H), 7.36 (d, J=8.3, 2H), 5.09 (s, 2H), 3.29 - 3.14 (m, 5H), 3.08 - 2.94 (m, 1 H), 2.82 - 2.70 (m, 1H), 2.49 - 2.25 (m, 5H), Rt[min] 1,62,
4-[2-(3H-Benzotriazol-5(R)-sulfinyl)-ethyl]-piperazin-1-carbonsäure 3-chloro-4-trifluoromethyl-benzyl ester (Ij) 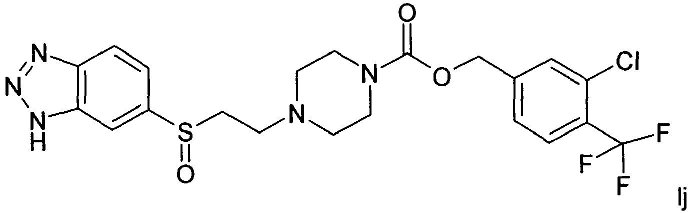 1H NMR (500 MHz, DMSO) δ = 16.01 (s, 0.5 H), 8.26 (s, 1H), 8.10 (d, J=8.6, 1H), 7.87 (d, J=8.1, 1H), 7.74 (d, J=8.6, 1H), 7.69 (s, 1H), 7.52 (d, J=8.1, 1H), 5.21 (m, 2H), 3.29 - 3.16 (m, 5H), 3.07 - 2.94 (m, 1 H), 2.84 - 2.70 (m, 1 H), 2.50 - 2.30 (m, 5H), Rt[min] 1,75,
4-[2-(3H-Benzotriazol-5(R)-sulfinyl)-ethyl]-piperazin-1-carbonsäure 3-fluoro-4-trifluoromethyl-benzyl ester 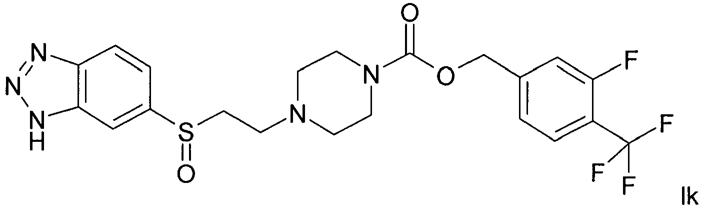 1 H NMR (500 MHz, DMSO) δ = 15.92 (m, 1 H), 8.25 (s, 1 H), 8.10 (d, J=8.6, 1 H), 7.80 (t, J=7.8, 1 H), 7.74 (d, J=8.7, 1 H), 7.48 (d, J=11.8, 1 H), 7.39 (d, J=8.0, 1 H), 5.17 (s, 2H), 3.28 - 3.15 (m, 5H), 3.08 - 2.96 (m, 1 H), 2.82 - 2.74 (m, 1 H), 2.49 - 2.25 (m, 5H), Rt[min] 1,69,
4-[2-(2-Oxo-2,3-dihydro-benzooxazol-6(R)-sulfinyl)-ethyl]-piperazin-1-carbonsäure 3-fluoro-5-trifluoromethyl-benzyl ester 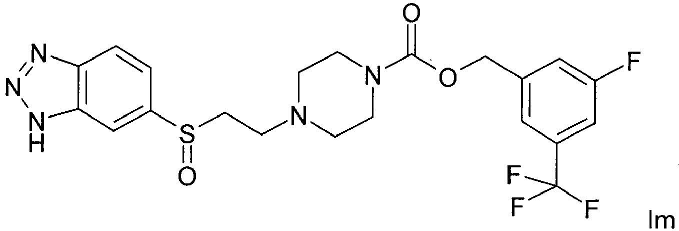 1 H NMR (500 MHz, DMSO) δ = 15.60 (s, 1 H), 8.24 (s, 1 H), 8.08 (d, J=8.6, 1 H), 7.72 (d, J=8.7, 1 H), 7.61 (d, J=8.6, 1 H), 7.58 - 7.48 (m, 2H), 5.16 (s, 2H), 3.36 - 3.14 (m, 5H), 3.07 - 2.93 (m, 1 H), 2.81 - 2.74 (m, 1 H), 2.49 - 2.27 (m, 5H), Rt[min] 1,69.

### Beispiel A: Autotaxin Test (Enzym Test)

### Testbeschreibung

Die Autotaxin Aktivität wird indirekt mit dem Amplex Red Reagenz gemessen. Hierbei wird Amplex Red als fluorgenischem Indikator für das entstandene H₂O₂ gemessen. Im Detail setzt Autotaxin das Substrat Lysophosphatidylcholin (LPC) zu Phosphocholin und Lysophosphatidylsäure (LPS) um. Nach dieser Umsetzung wird das Phosphocholin mit alkalischer Phosphatase zu inorganischem Phosphat und Cholin ungesetzt. Im nächsten Schritt wird Cholin durch Choline-Oxidase zu Betain oxidiert, wobei H₂O₂ entsteht. H₂O₂ reagiert in Gegenwart von Peroxidase (Horseradish peroxidase) mit dem Amplex Red Reagenz in eine 1:1 Stöchiometrie und bildet das hochfluoreszente Resorufin. Die Fluoreszenz wird in einem reaktionsabhängigen kinetischen Modus gemessen, damit dass fluoreszente Signale möglicher anderer fluoreszenter Stoffe, die nicht an der Reaktion beteiligt sind, herauskorrigiert werden kann.

### Testausführung

1,5 µl einer Standardlösung oder der Testsubstanzen (Substanzen mit dem Namen A(n)) in individuellen Konzentrationen gelöst in 20mM Hepes pH 7.2 mit maximal 7.7% DMSO werden zusammen mit 10 µl (16 ng) hochgereinigten recombinanten Autotaxin in einer schwarzen mit 384 Vertiefungen versehenen Mikrotiterplatte für 30 min bei 22°C vorinkubiert. Danach wird die Reaktion durch Zugabe von 5µl L-α-Lysophosphatidylcholin (LPC) gestartet, wobei die Endkonzentration von LPC 75 µM beträgt. Die Mischung wird 90 min. bei 37°C inkubiert. Nach der Inkubation wird Amplex Red Reagenz, Peroxidase (Horseradish peroxidase) und Cholin-Oxidase hinzugefügt und sofort die Fluoreszenz bei 612 nm bei einer Anregung von 485 nm in einem "Tecan Ultra multimode" Lesegerät gemessen. Die Aktivität von Autotaxin wird indirekt über den Nachweis des anfallenden H₂O₂ errechnet.

### Material:

| | |
|---|---|
| Microtiterplatte: | PS-Microplate, 384 Vertiefungen, kleines Volumen, schwarz Corning, Cat#3677 |
| Protein: | Recombinantes Autotaxin (Baculovirale Hi5 Expression) |
| Substrat: | L-α-Lysophosphatidylcholin (Hühnerei)); Avanti Polar Lipids # 830071 P |
| Standard: | C14 LPA, Avanti Polar Lipids, Cat# 857120P |
| Nachweis Reagenz: | Amplex Red Reagenz ; Invitrogen # A12222; gelöst in 1.923 ml of DMSO Peroxidase Type VI-A (horseradish) von Sigma # P6782; gelöst in 7,45 ml Test Puffer, Choline-Oxidase ; Sigma # C5896; gelöst in 2,47 ml Test Puffer |
| Nachweis Reagenz Mix: | 1:100 Verdünnung von Amplex Red Reagenz in Test Puffer |
| Test Puffer: | 200 mM Tris-HCl, Merck, Cat # 1.08219, pH 7.9, 0.1 % BSA, lipidfrei, Roche Cat#775835 |

Die IC50-Werte aller beschriebenen Verbindungen sind < 1µM in dieseM Assay.

Die nachfolgenden Beispiele betreffen Arzneimittel:

### Beispiel B: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel C: Suppositorien

Man schmilzt ein Gemisch von 20 g eines Wirkstoffes der Formel I mit 100 g Sojalecithin und 1400 g Kakaobutter, gießt in Formen und lässt erkalten. Jedes Suppositorium enthält 20 mg Wirkstoff.

### Beispiel D: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel E: Salbe

Man mischt 500 mg eines Wirkstoffes der Formel I mit 99,5 g Vaseline unter aseptischen Bedingungen.

### Beispiel F: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpresst, derart, dass jede Tablette 10 mg Wirkstoff enthält.

### Beispiel G: Dragees

Analog Beispiel E werden Tabletten gepresst, die anschließend in üblicher Weise mit einem Überzug aus Saccharose, Kartoffelstärke, Talk, Tragant und Farbstoff überzogen werden.

### Beispiel H: Kapseln

2 kg Wirkstoff der Formel I werden in üblicher Weise in Hartgelatinekapseln gefüllt, so dass jede Kapsel 20 mg des Wirkstoffs enthält.

### Bespiel I: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Die Verbindung la bis Im 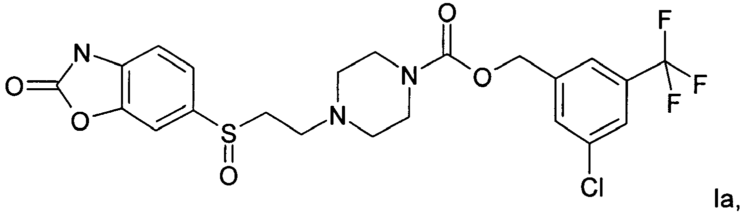 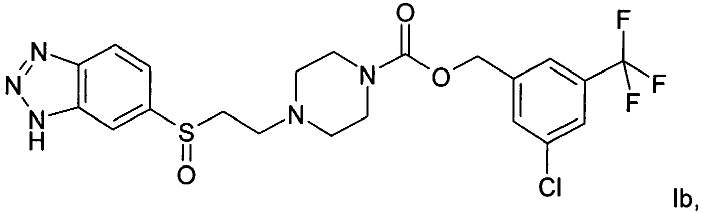 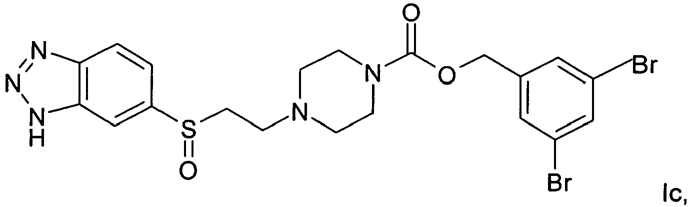 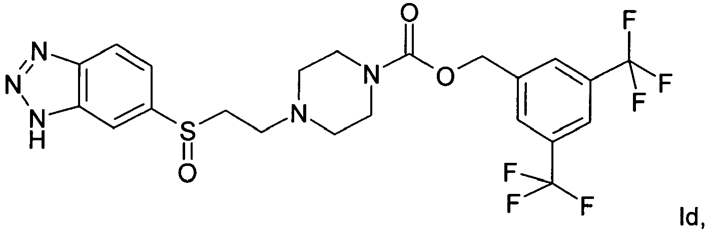 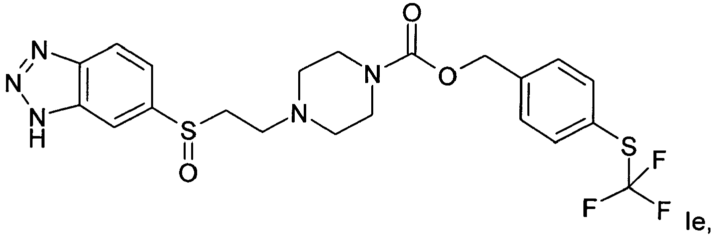 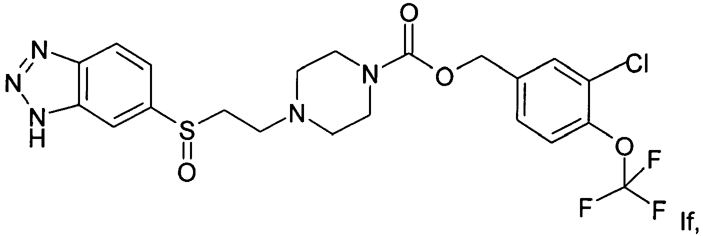 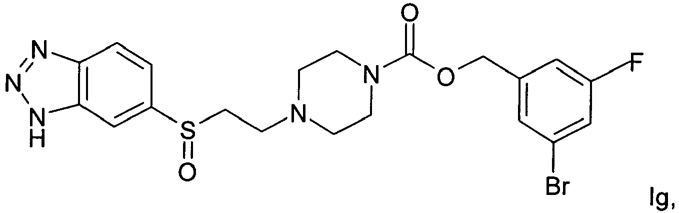 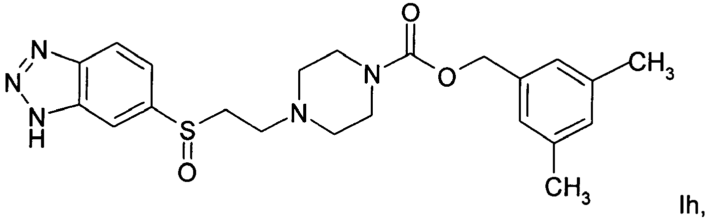 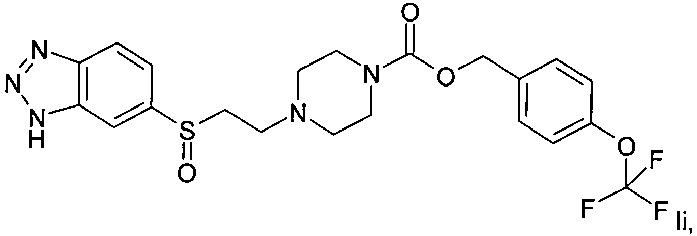 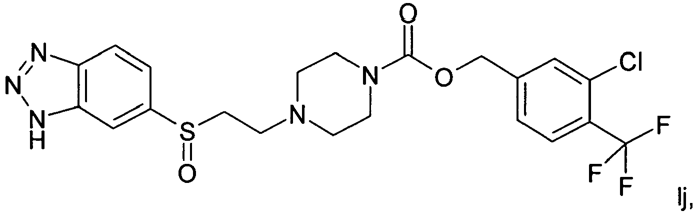 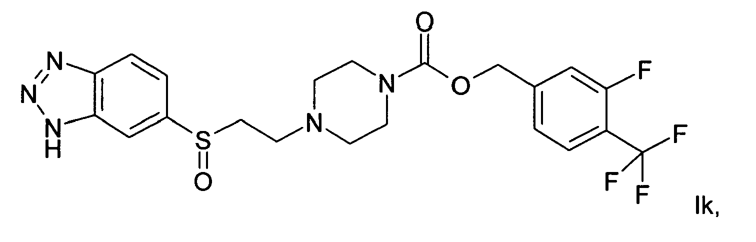 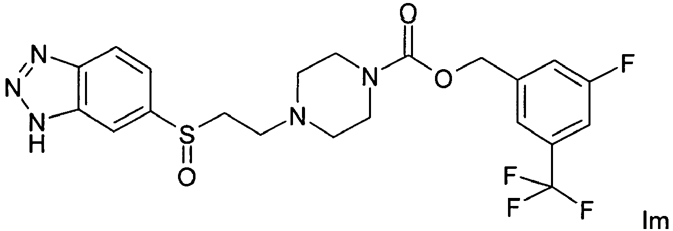 sowie
seine pharmazeutisch verwendbaren Salze, Solvate, Enatiomere, Tautomere und Stereoisomere, einschließlich deren Mischungen in allen Verhältnissen.

2. Arzneimittel, enthaltend mindestens eine Verbindung nach Anspruch 1 und/oder seine pharmazeutisch verwendbaren Salze, Solvate, Enantiomere, Tautomere und Stereoisomere, einschließlich deren Mischungen in allen Verhältnissen, sowie gegebenenfalls Trägerund/oder Hilfsstoffe.

3. Verwendung der Verbindungen ausgewählt aus der Gruppe 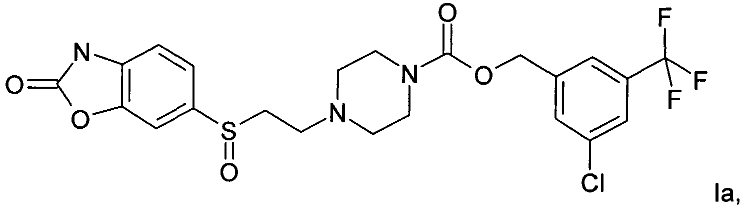 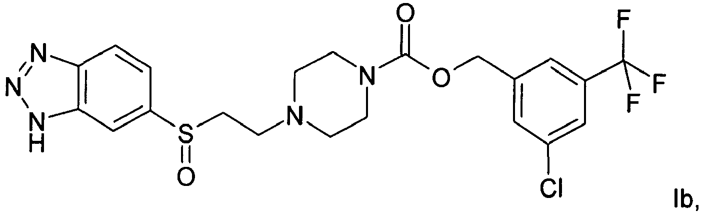 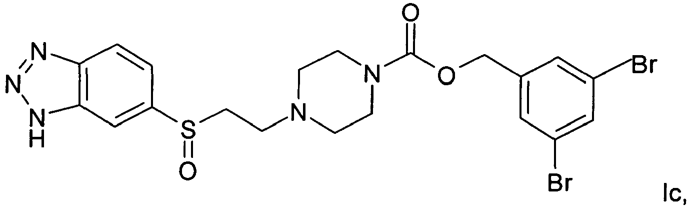 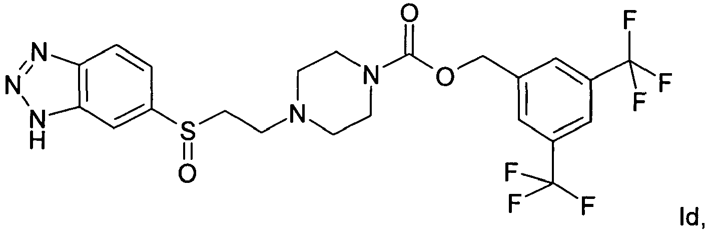 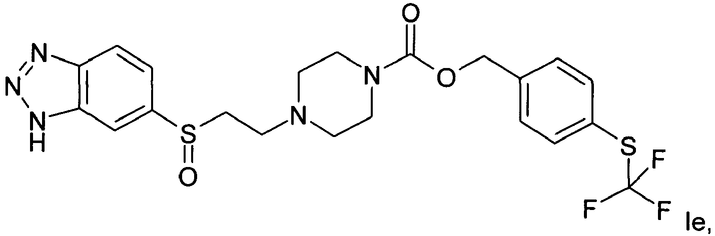 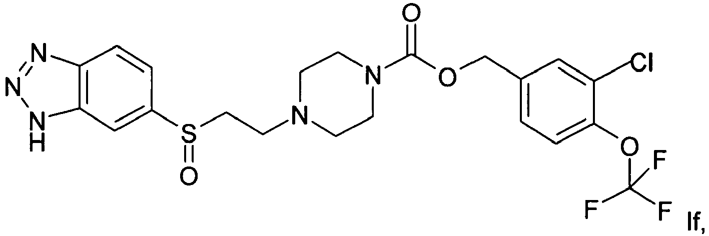 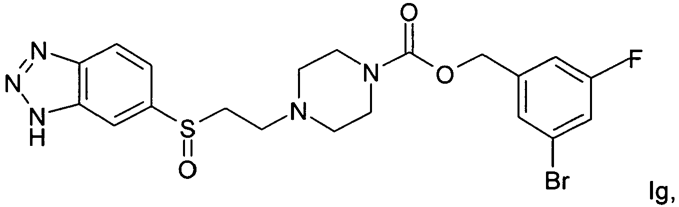 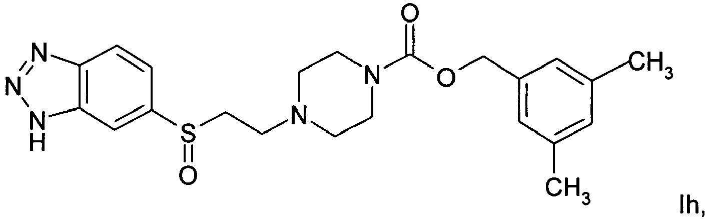 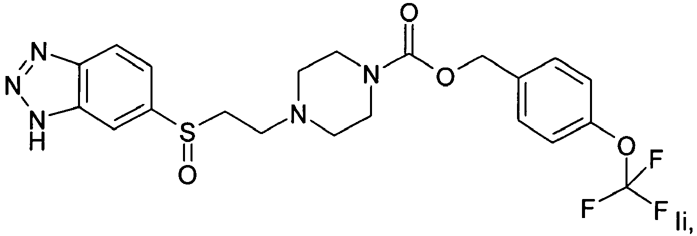 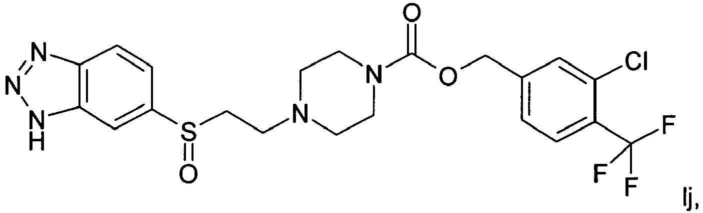 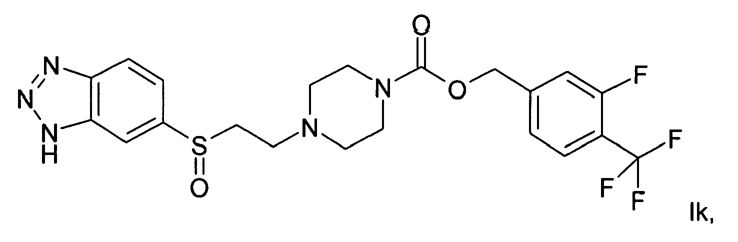 und /oder 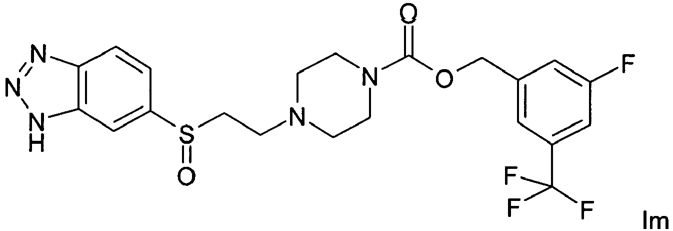 sowie seiner pharmazeutisch verwendbaren Salze, Solvate, Tautomere und Stereoisomere, einschließlich deren Mischungen in allen Verhältnissen, zur Herstellung eines Arzneimittels zur Behandlung oder Vorbeugung von Tumorerkrankungen.

4. Verwendung nach Anspruch 3, wobei es sich bei den Tumorerkrankungen um Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiosarkom, Rhabdomyosarkom, Kolonkarzinom, Pankreaskrebs, Brustkrebs, Ovarkrebs, Prostatakrebs, Plattenzellkarzinom, Basalzellkarzinom, Adenokarzinom, Schweißdrüsenkarzinom, Talgdrüsenkarzinom, Papillarkarzinom, Papillaradenokarzinomen, Cystadenokarzinomen, Knochenmarkkarzinom, bronchogenem Karzinom, Nierenzellkarzinom, Hepatom, Gallengangkarzinom, Chorionkarzinom, Seminom, embryonalem Karzinom, Wilms-Tumor, Cervix-Krebs, Hodentumor, Lungenkarzinom, kleinzelligem Lungenkarzinom, Blasenkarzinom, Epithelkarzinom, Gliom, Astrocytom, Medulloblastom, Kraniopharyngiom, Ependymom, Pinealom, Hämangioblastom, akustischem Neurom, Oligodendrogliom, Meningiom, Melanom, Neuroblastom, Retinoblastom, Leukämie, Lymphom, multiplem Myelom, Waldenströms Makroglobulinämie und Schwere-Kettenerkrankung handelt.

5. Arzneimittel enthaltend eine oder mehrere Verbindungen gemäß Anspruch 1 und/oder seine pharmazeutisch verwendbaren Salze, Solvate, Enantiomere, Tautomere und Stereoisomere, einschließlich deren Mischungen in allen Verhältnissen,
und mindestens einen weiteren Arzneimittelwirkstoff.

6. Set (Kit), bestehend aus getrennten Packungen
(a) einer wirksamen Menge der Verbindungen 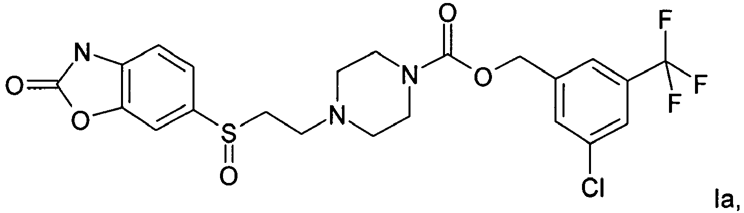 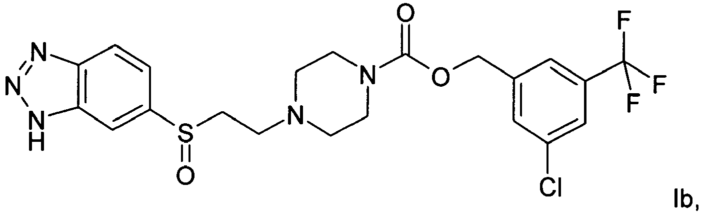 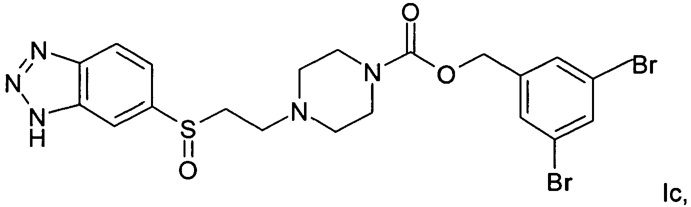 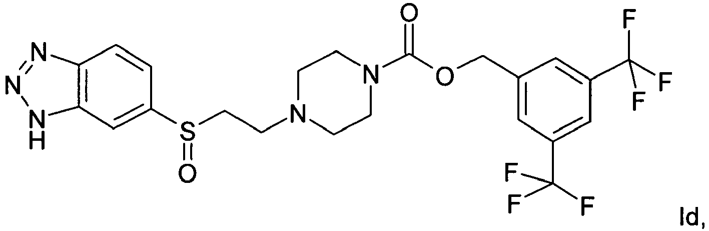 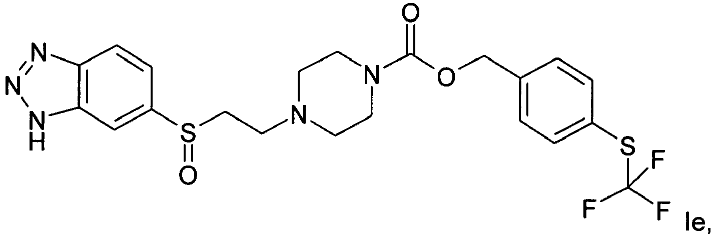 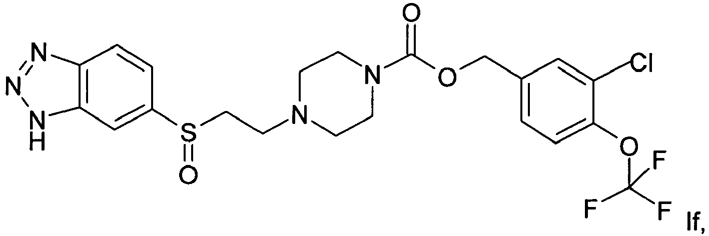 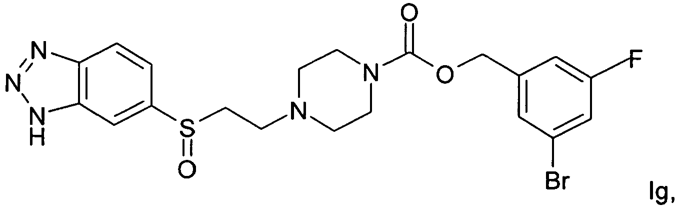 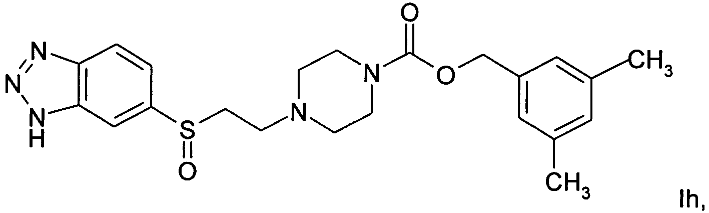 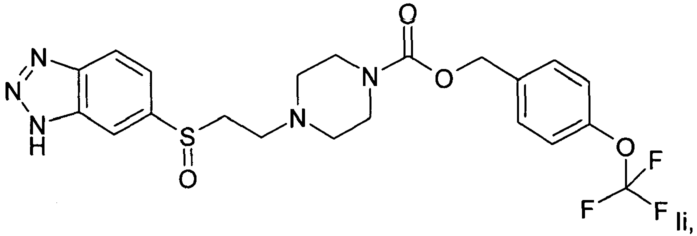 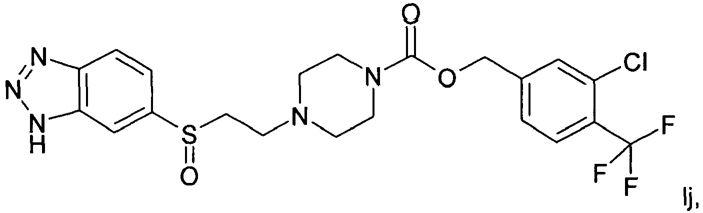 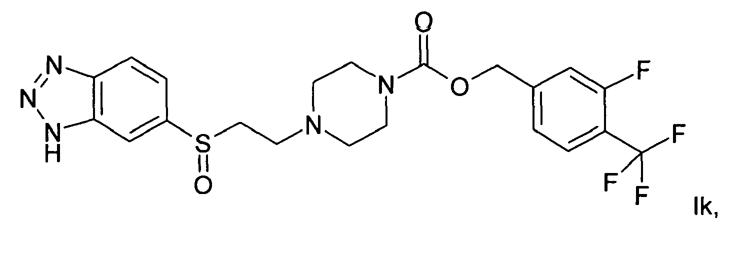 und/oder 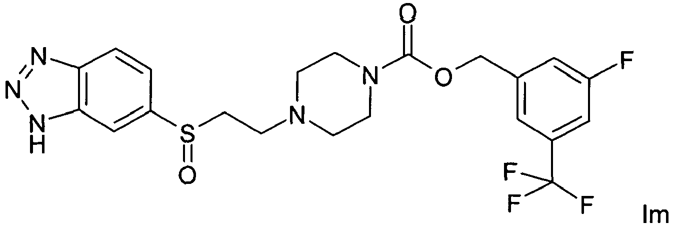 und/oder seiner pharmazeutisch verwendbaren Salze, Solvate, Enantiomere, Tautomere und Stereoisomere, einschließlich deren Mischungen in allen Verhältnissen,
und
(b) einer wirksamen Menge eines weiteren Arzneimittelswirkstoffs.

## Claims

1. Compounds la to Im 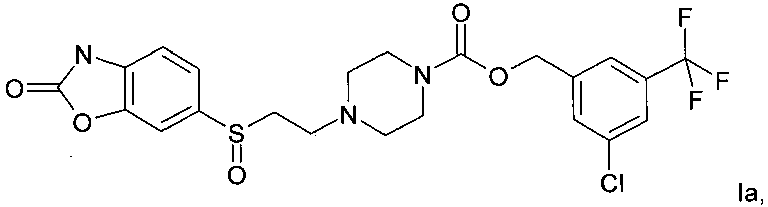 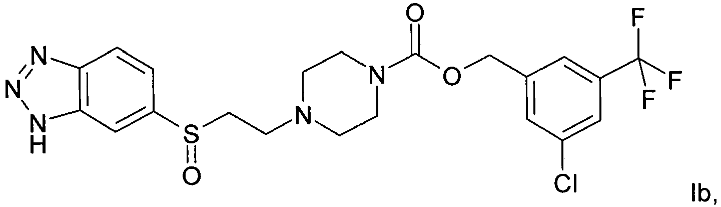 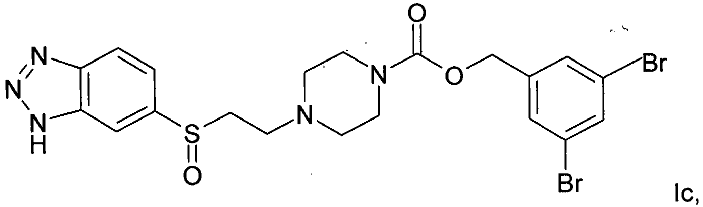 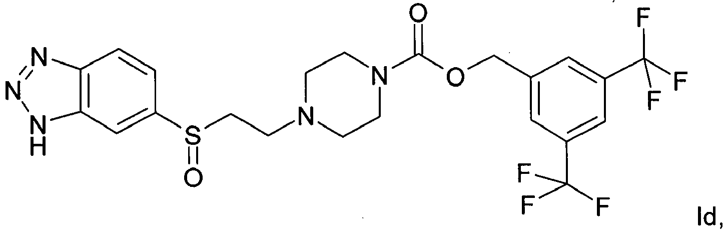 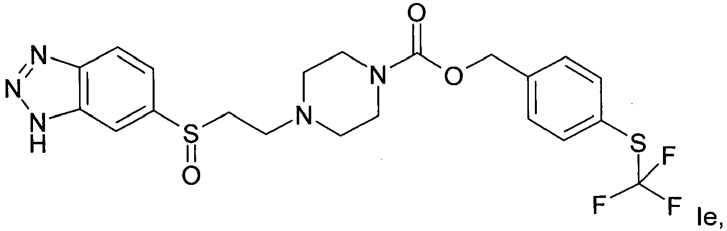 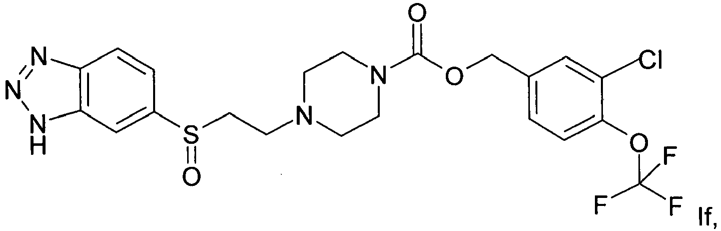 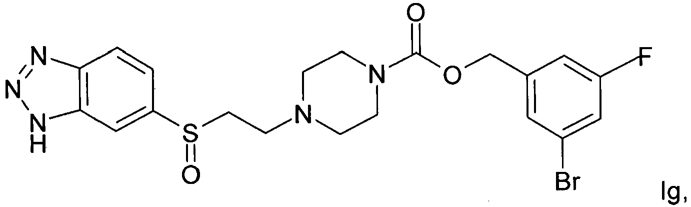 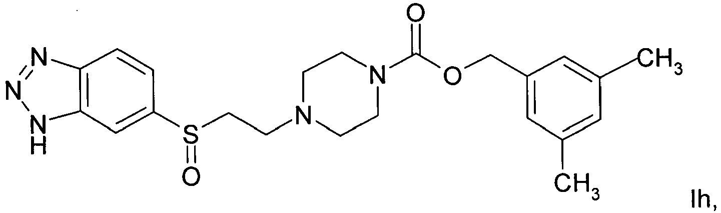 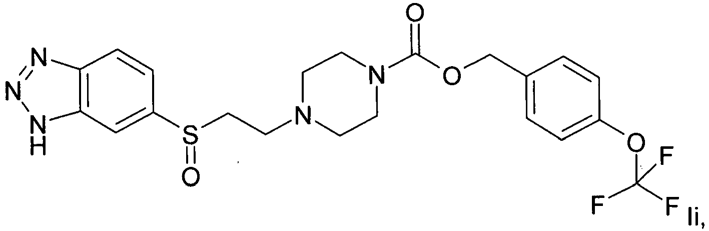 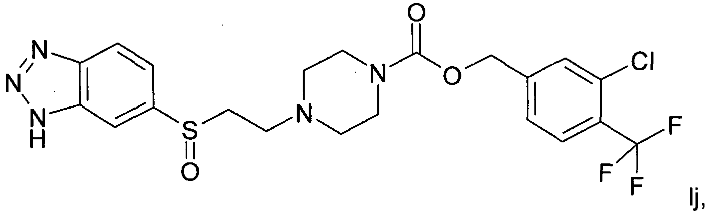 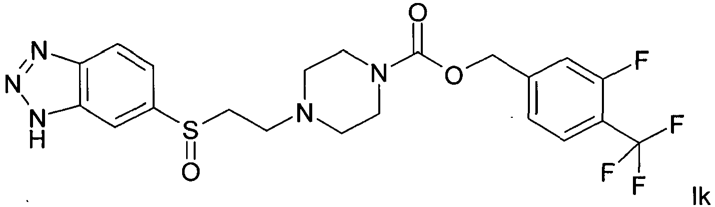 and 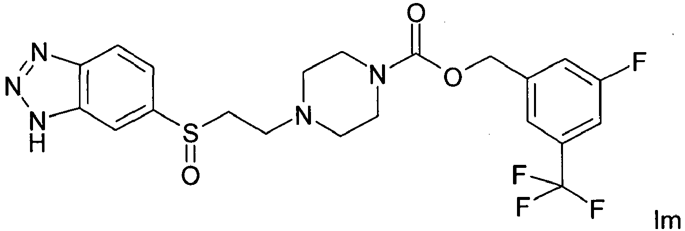 and pharmaceutically usable salts, solvates, enantiomers, tautomers and stereoisomers thereof, including mixtures thereof in all ratios.

2. Medicaments comprising at least one compound according to Claim 1 and/or pharmaceutically usable salts, solvates, enantiomers, tautomers and stereoisomers thereof, including mixtures thereof in all ratios, and optionally excipients and/or adjuvants.

3. Use of the compounds selected from the group 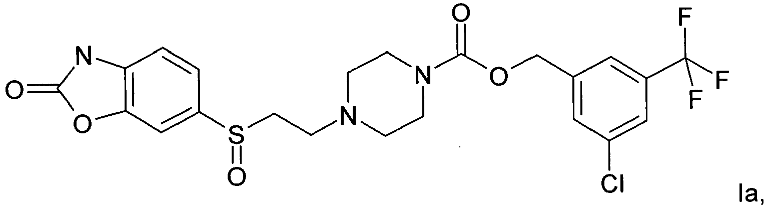 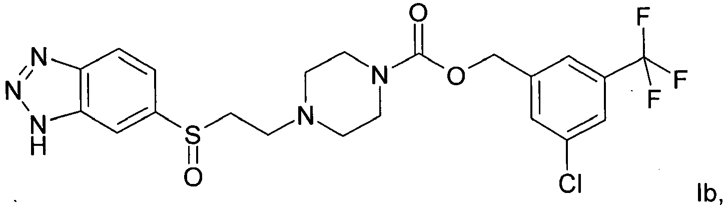 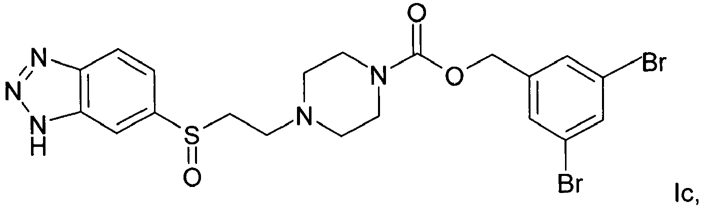 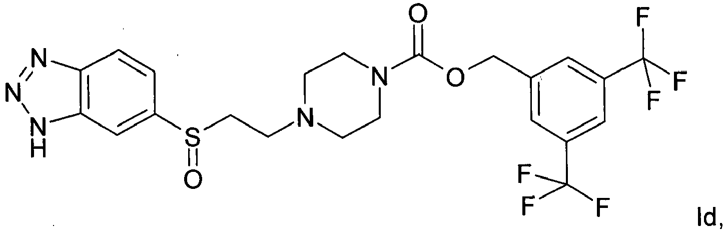 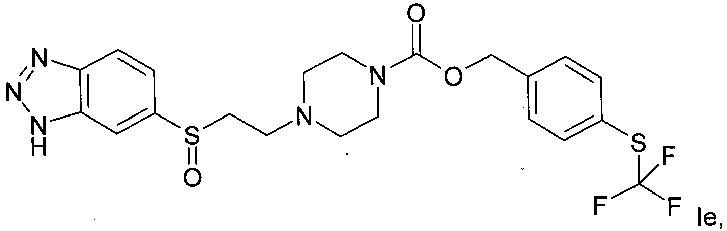 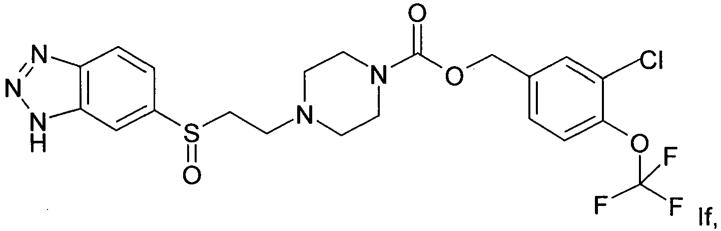 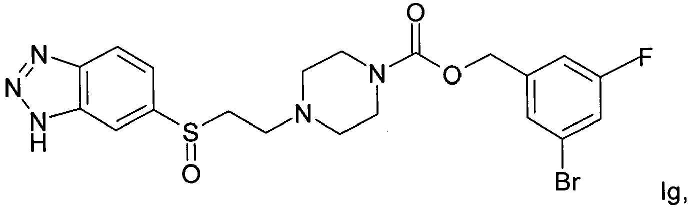 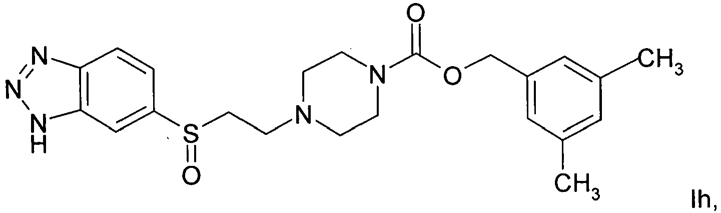 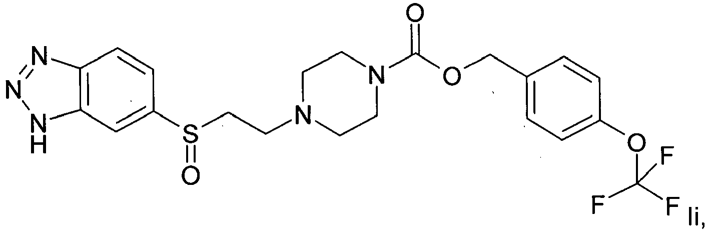 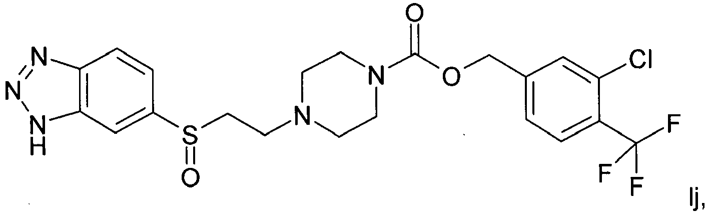 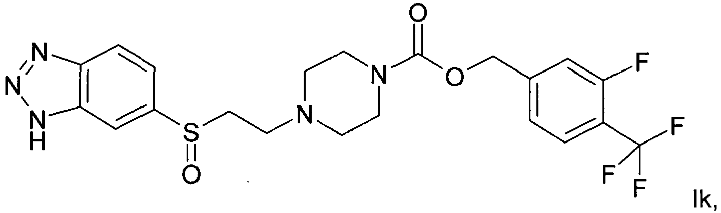 and/or 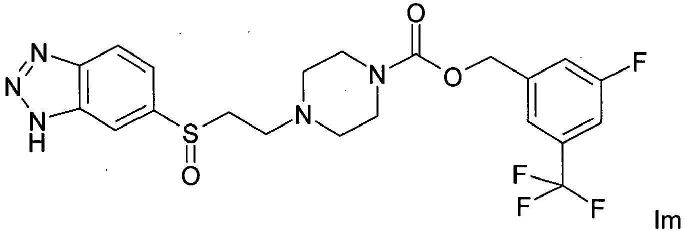 and pharmaceutically usable salts, solvates, tautomers and stereoisomers thereof, including mixtures thereof in all ratios, for the preparation of a medicament for the treatment or prevention of tumour diseases.

4. Use according to Claim 3, where the tumour diseases are fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumour, leiosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinomas, bone marrow carcinoma, bronchogenic carcinoma, renal cell carcinoma, hepatoma, bile duct carcinoma, choriocarcinoma, seminoma, embryonic carcinoma, Wilms' tumour, cervical cancer, testicular tumour, lung carcinoma, small-cell lung carcinoma, bladder carcinoma, epithelial carcinoma, glioma, astrocytoma, medulloblastoma, craniopharyngioma, ependymoma, pinealoma, haemangioblastoma, acoustic neuroma, oligodendroglioma, meningioma, melanoma, neuroblastoma, retinoblastoma, leukaemia, lymphoma, multiple myeloma, Waldenström's macroglobulinaemia and heavy chain disease.

5. Medicaments comprising one or more compounds according to Claim 1 and/or pharmaceutically usable salts, solvates, enantiomers, tautomers and stereoisomers thereof, including mixtures thereof in all ratios,
and at least one further medicament active compound.

6. Set (kit) consisting of separate packs of
(a) an effective amount of the compounds 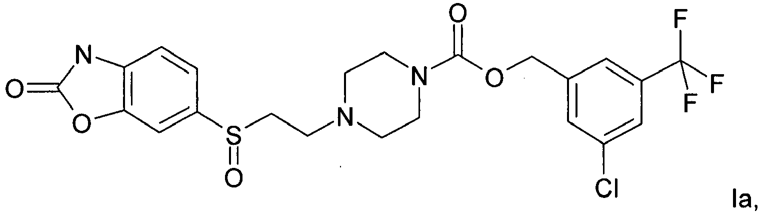 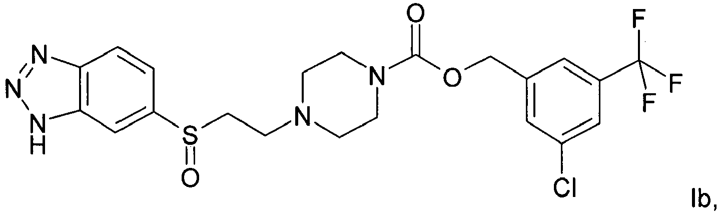 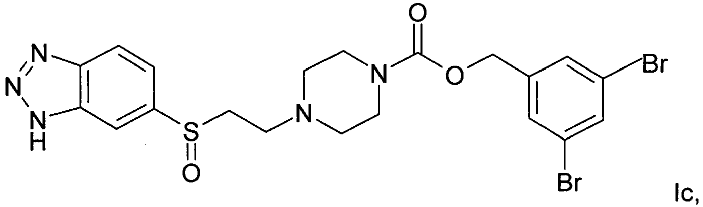 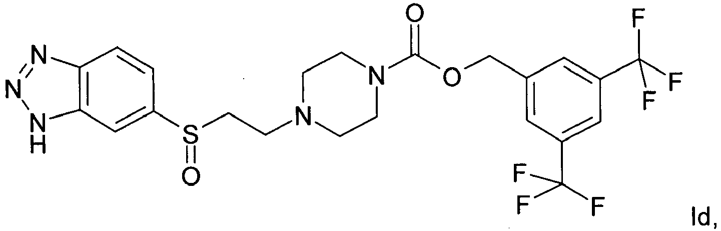 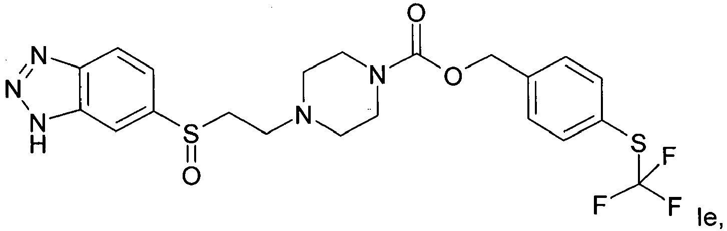 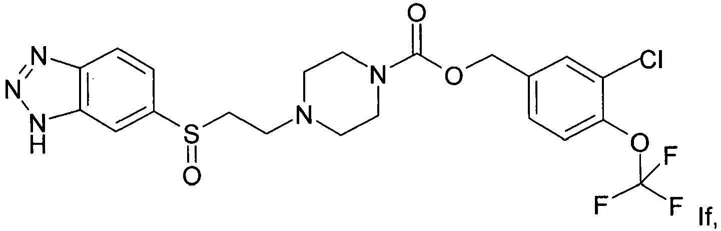 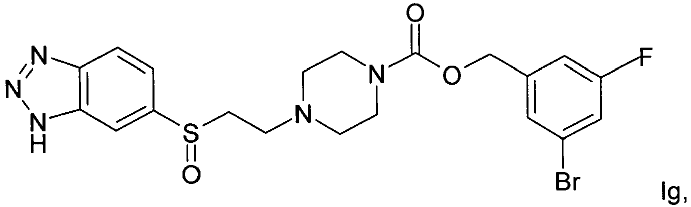 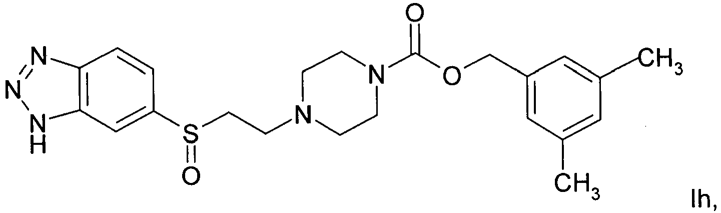 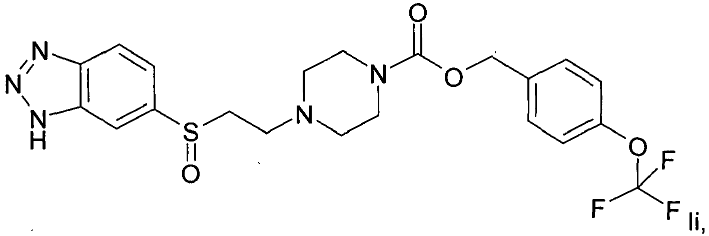 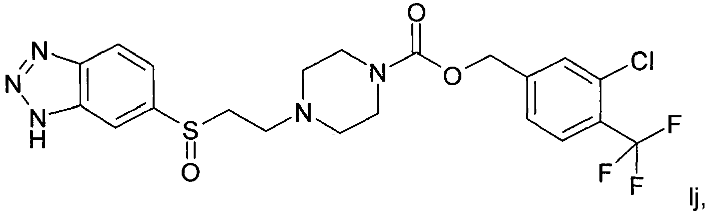 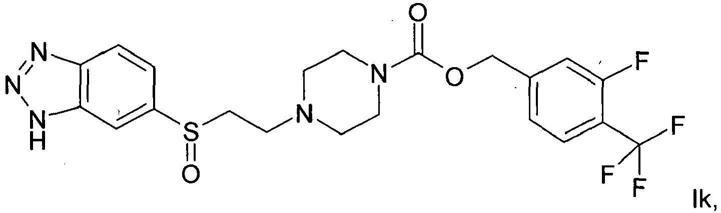 and/or 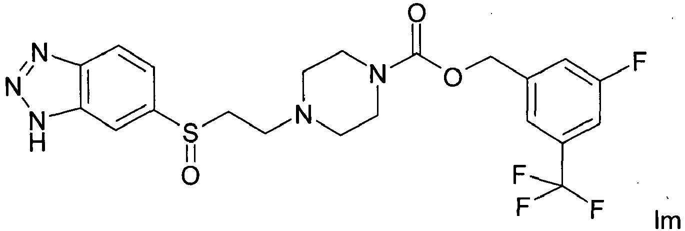 and/or pharmaceutically usable salts, solvates, enantiomers, tautomers and stereoisomers thereof, including mixtures thereof in all ratios,
and
(b) an effective amount of a further medicament active compound.

## Revendications

1. Composés la à lm 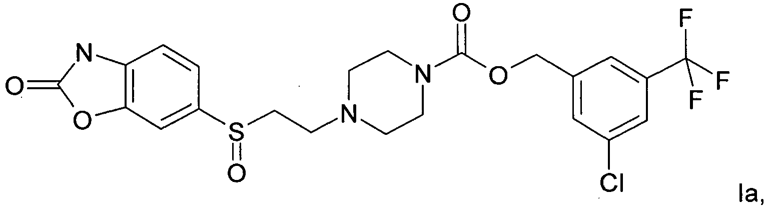 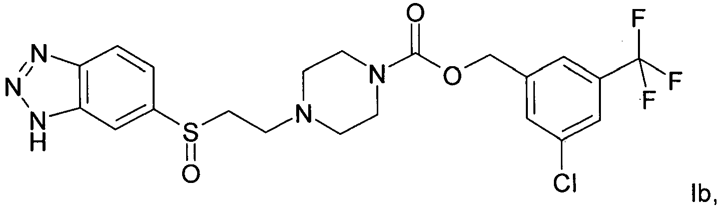 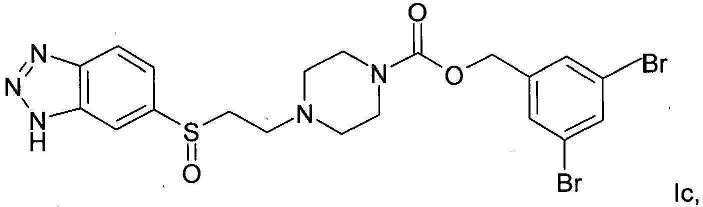 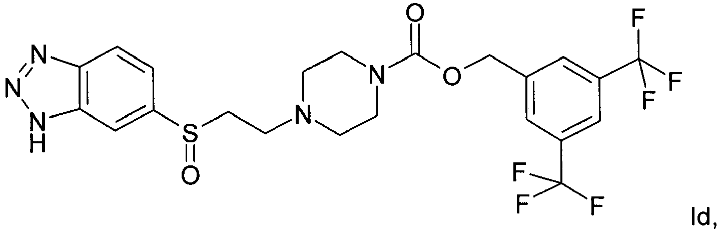 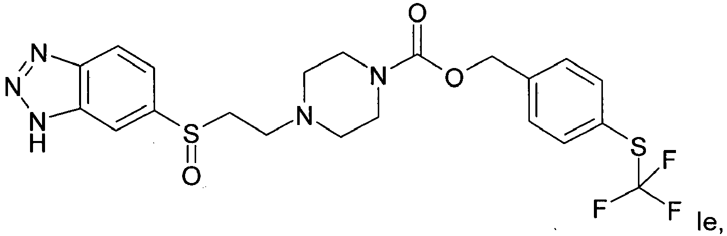 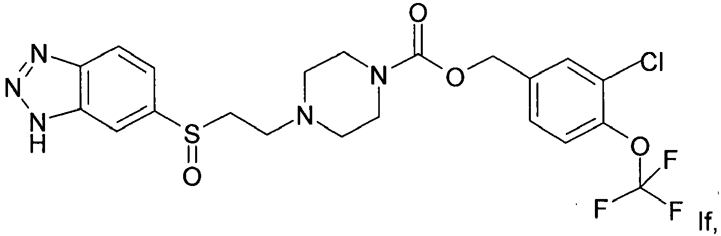 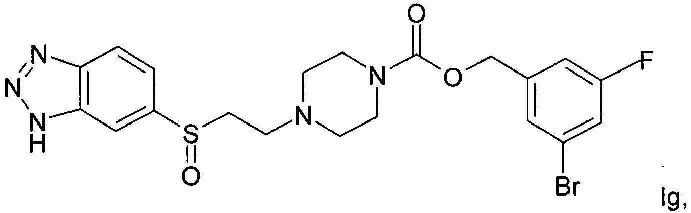 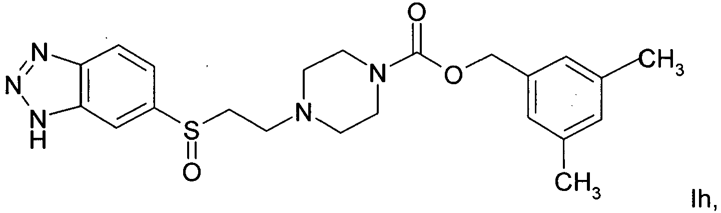 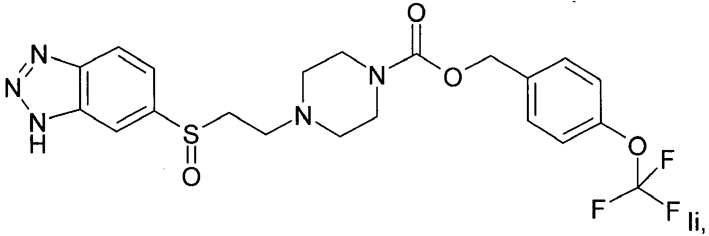 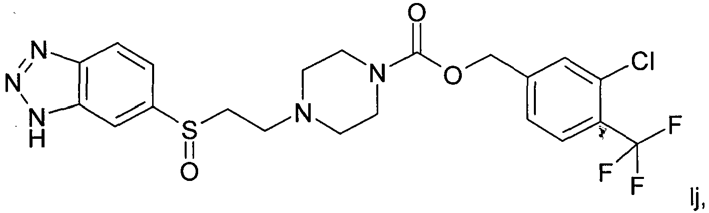 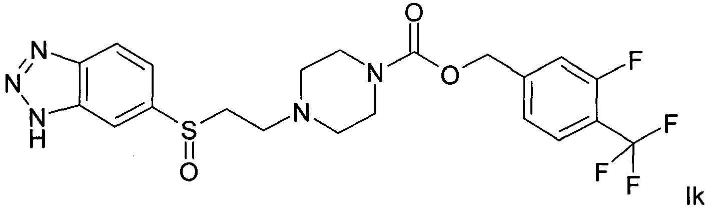 and 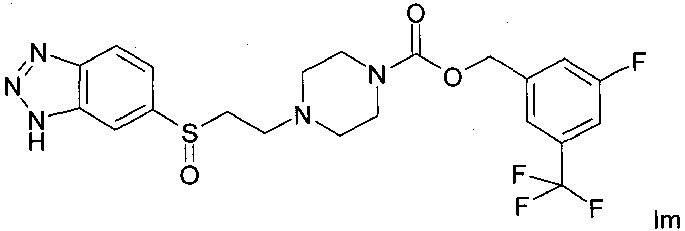 ainsi que les sels, solvates, énantiomères, tautomères et stéréoisomères pharmaceutiquement utilisables de ceux-ci, y compris des mélanges de ceux-ci en toutes proportions.

2. Médicaments comprenant au moins un composé selon la revendication 1 et/ou des sels, solvates, énantiomères, tautomères et stéréoisomères pharmaceutiquement utilisables de celui-ci, y compris des mélanges de ceux-ci en toutes proportions, et éventuellement des excipients et/ou des adjuvants.

3. Utilisation des composés choisis dans le groupe constitué par 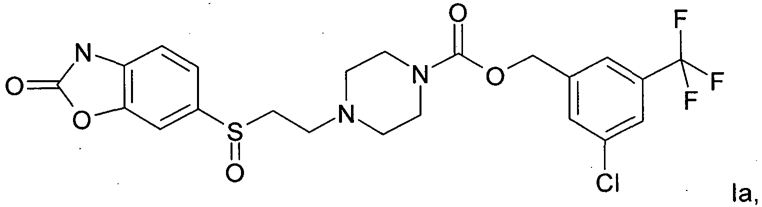 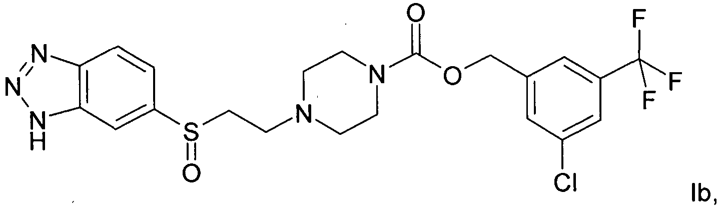 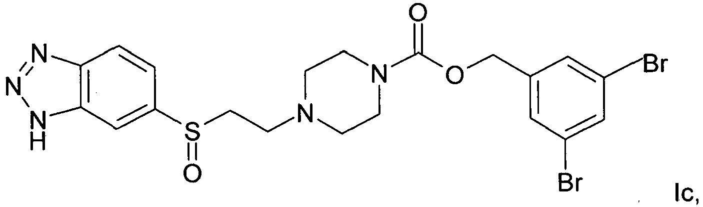 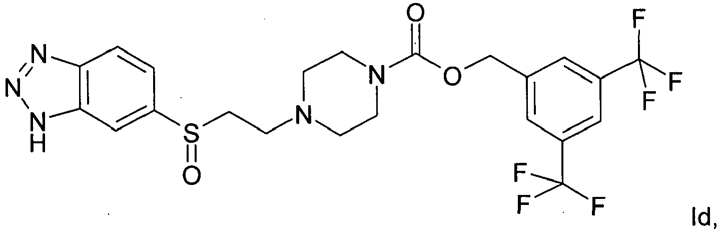 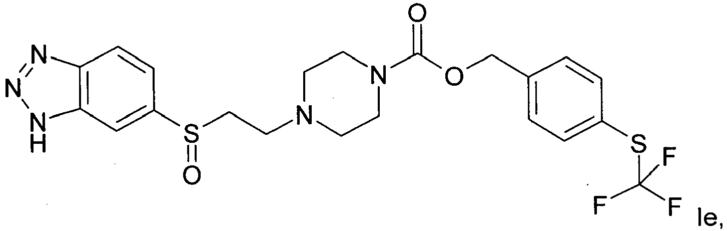 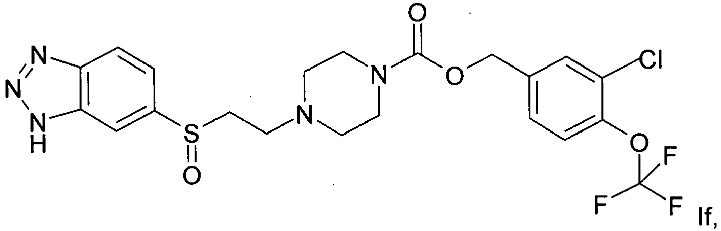 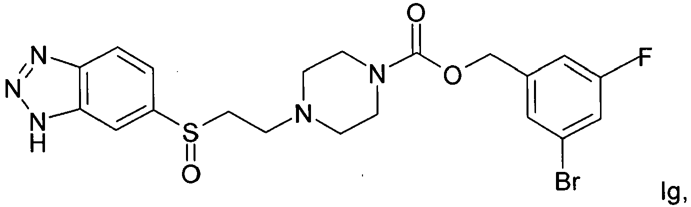 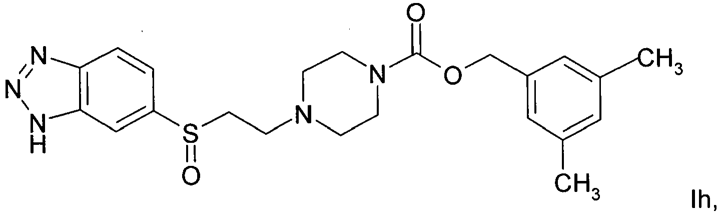 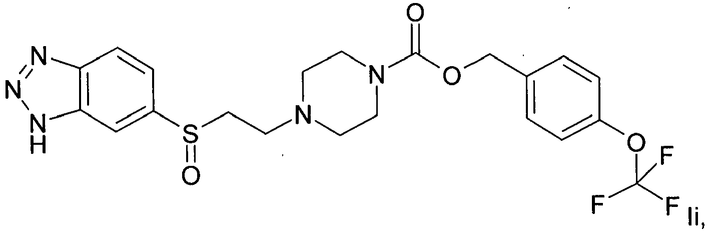 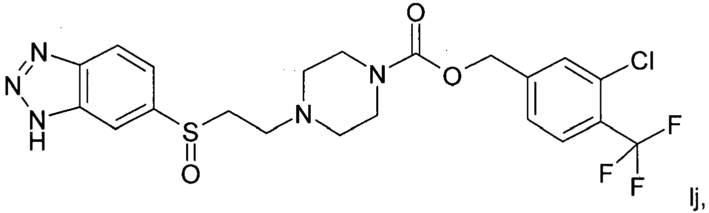 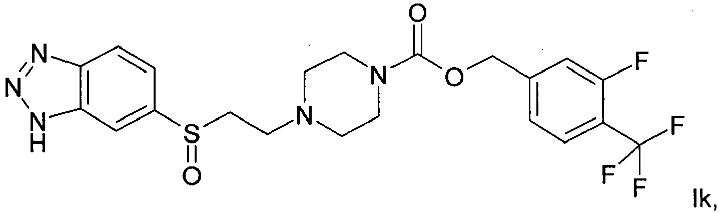 et/ou 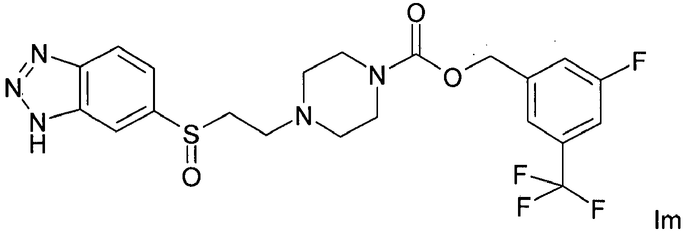 ainsi que les sels, solvates, tautomères et stéréoisomères pharmaceutiquement utilisables de ceux-ci, y compris des mélanges de ceux-ci en toutes proportions, pour la préparation d'un médicament destiné au traitement ou à la prévention de maladies tumorales.

4. Utilisation selon la revendication 3, dans laquelle les maladies tumorales sont le fibrosarcome, le myxosarcome, le liposarcome, le chondrosarcome, le sarcome ostéogénique, le chordome, l'angiosarcome, l'endothéliosarcome, le lymphangiosarcome, le lymphangioendothéliosarcome, le synoviome, le mésothéliome, le sarcome d'Ewing, le léiomyosarcome, le rhabdomyosarcome, le carcinome du côlon, le cancer du pancréas, le cancer du sein, le cancer de l'ovaire, le cancer de la prostate, le carcinome des cellules squameuses, l'épithélioma basocellulaire, l'adénocarcinome, l'hidradénome, le carcinome des glandes sébacées, le carcinome papillaire, les adénocarcinomes papillaires, les cystadénocarcinomes, le carcinome de la moelle osseuse, le carcinome bronchogénique, le carcinome des cellules rénales, l'hépatome, le carcinome du canal biliaire, le chorio-épithéliome, le séminome, le carcinome embryonnaire, le néphroblastome, le cancer du col de l'utérus, les tumeurs testiculaires, le carcinome du poumon, le carcinome du poumon à petites cellules, le carcinome de la vessie, le carcinome épithélial, le gliome, l'astrocytome, le médulloblastome, le craniopharyngiome, l'épendymome, le pinéalome, l'hémangioblastome, le neurinome de l'acoustique, l'oligodendrogliome, le méningiome, le mélanome, le neuroblastome, le rétinoblastome, la leucémie, le lymphome, le myélome multiple, la macroglobulinémie de Waldenström et la maladie des chaînes lourdes.

5. Médicaments comprenant un ou plusieurs composés selon la revendication 1 et/ou des sels, solvates, énantiomères, tautomères et stéréoisomères pharmaceutiquement utilisables de ceux-ci, y compris des mélanges de ceux-ci en toutes proportions,
et au moins un autre composé actif médicamenteux.

6. Ensemble (kit) constitué de conditionnements séparés
(a) d'une quantité efficace des composés 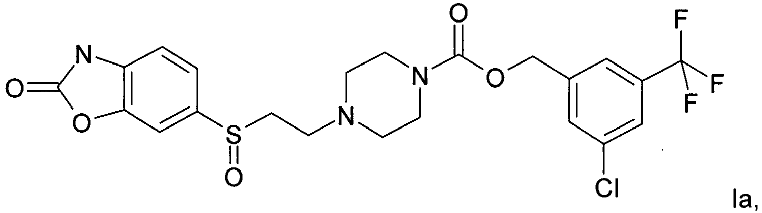 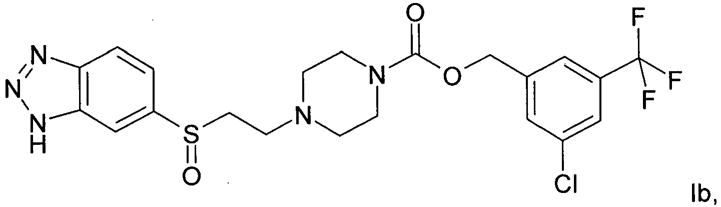 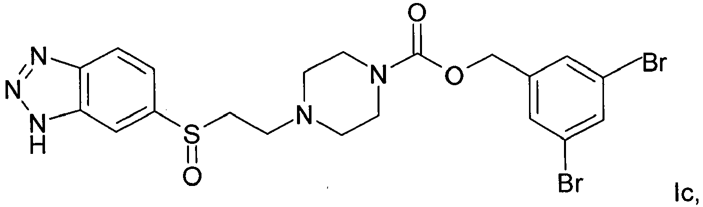 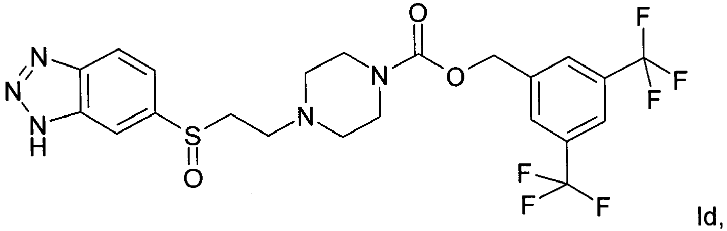 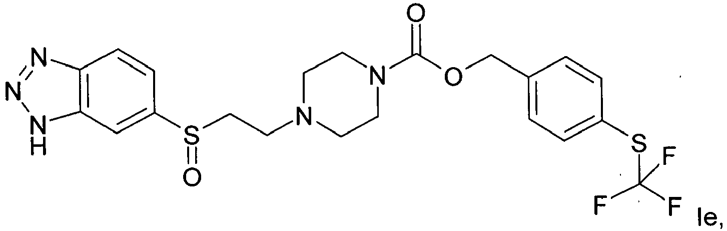 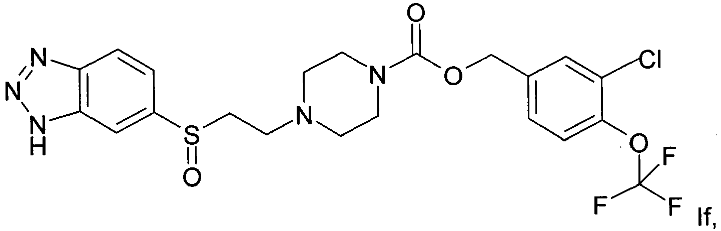 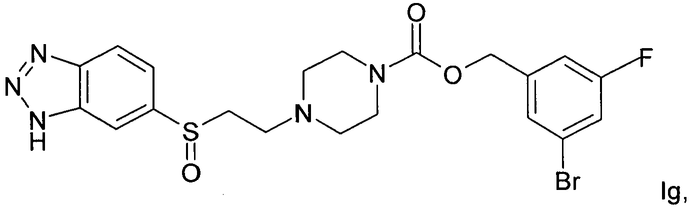 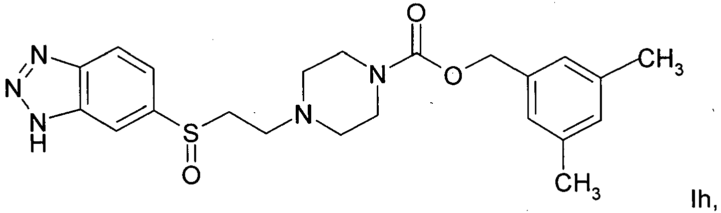 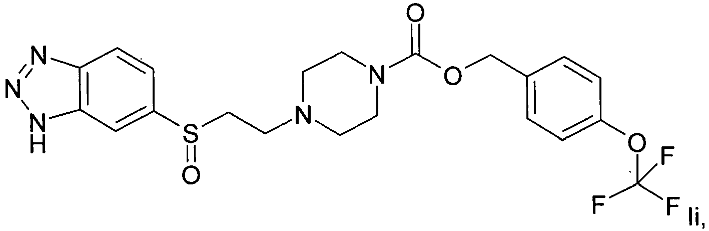 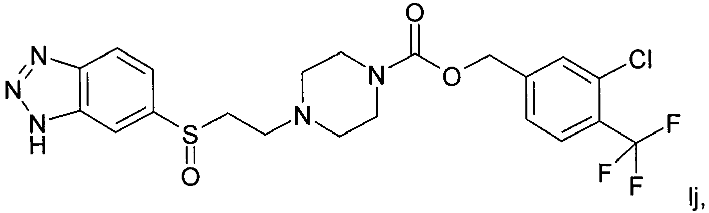 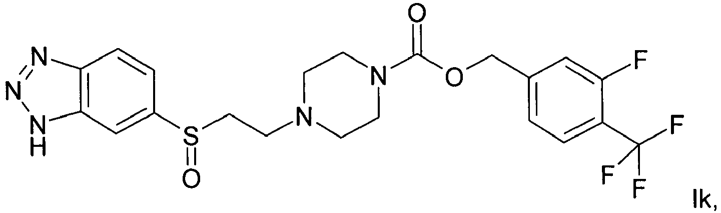 et/ou 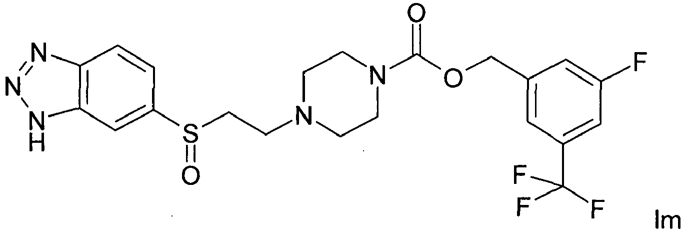 et/ou de sels, solvates, énantiomères, tautomères et stéréoisomères pharmaceutiquement utilisables de ceux-ci, y compris des mélanges de ceux-ci en toutes proportions,
et
(b) d'une quantité efficace d'un autre composé actif médicamenteux.
